# EUROPEAN PATENT APPLICATION

(11) **EP 4 190 310 A1**
(43) Date of publication of application: **07.06.2023**
(21) Application number: 21851412.3
(22) Date of filing: 29.07.2021
(51) Int. Cl.: A61K 9/08, A61K 47/26, A61K 47/10, A61K 47/18, A61K 47/22, A61K 38/00, A61K 38/17, A61K 9/00, A61P 27/02

(54) **STABLE PHARMACEUTICAL PREPARATION**

(30) Priority: 31.07.2020 KR 20200096434
(71) Applicant: Celltrion, Inc., Incheon 22014 (KR)
(72) Inventor: KIM, Su Jung, Incheon 22014 (KR); KIM, Kwang Woo, Incheon 22014 (KR); ROH, Ji Won, Incheon 22014 (KR); SHIN, Yeon Kyeong, Incheon 22014 (KR); OH, Jun Seok, Incheon 22014 (KR); LEE, Jae Bin, Incheon 22014 (KR); HAN, Won Yong, Incheon 22014 (KR)
(74) Representative: Cabinet Laurent & Charras
(86) International application number: PCT/KR2021/009870
(87) International publication number: WO 2022/025660

(57) **Abstract**

A stable pharmaceutical formulation according to the present disclosure contains: a recombinant fusion protein; a surfactant; a sugar or a derivative thereof; a buffer; and an isotonic agent. The stable pharmaceutical formulation according to the present disclosure has low viscosity while containing the recombinant fusion protein, may maintain excellent stability under long-term storage conditions, accelerated conditions and stress conditions, and may be administered intraocularly.

## Description

### CROSS-REFERENCE TO RELATED APPLICATION(S)

This application claims priority to Korean Patent Application No. 10-2020-0096434, filed on July 31, 2020, the disclosure of which is incorporated herein by reference in its entirety.

### BACKGROUND

### Technical Field

The present disclosure relates to a pharmaceutical formulation, which is capable of stably preserving a drug capable of inhibiting vascular endothelial growth factor (VEGF) and may be intraocularly administered.

### Description of the Related Art

Since release of vascular endothelial growth factor (VEGF) contributes to increased vascular permeability in the eye and inappropriate new blood vessel growth, it may cause neovascular (wet) age-related macular degeneration, visual impairment due to retinal vein occlusion (central retinal vein occlusion or branch retinal vein occlusion) macular edema, visual impairment due to diabetic macular edema, and visual impairment due to choroidal neovascularization caused by pathologic myopia. Thus, inhibiting VEGF is an effective method for treating ophthalmic diseases associated with angiogenesis.

The generic name for a VEGF inhibitor drug is aflibercept which is a recombinant fusion protein capable of inhibiting type A VEGF, type B VEGF, and placental growth factor (PIGF). A pharmaceutical formulation containing this drug should be administered by intraocular injection, and such administration should be performed directly by a qualified physician with experience in intraocular administration.

### SUMMARY

For long-term storage of a VEGF inhibitor drug, the present inventors developed a formulation containing histidine, trehalose, and polysorbate 20 as stabilizers, and added sodium chloride as an isotonic agent to the formulation so as to have an osmotic pressure similar to that in the eye for the purpose of reducing pain during administration. However, the present inventors confirmed that the stability of the drug decreased in the presence of sodium chloride as an isotonic agent. To overcome this problem, the present inventors applied sodium chloride at a concentration capable of maintaining an appropriate osmotic pressure.

The formulation according to the present disclosure exhibited improved results in terms of drug stabilization compared to a liquid aflibercept formulation disclosed in Korean Patent Application Publication No. 10-2009-0018807, and the improved level was directly compared and evaluated through an experimental example (Experimental Example 1).

Therefore, the present inventors have finally confirmed the superiority of a stable pharmaceutical formulation containing the VEGF inhibitor drug aflibercept, which is the formulation of the present disclosure.

Accordingly, an object of the present disclosure is to provide a pharmaceutical formulation which is stable during long-term storage while containing the VEGF inhibitor drug aflibercept.

Another object of the present disclosure is to provide a pharmaceutical formulation having excellent long-term storage stability based on excellent stability under accelerated conditions and stress conditions.

Still another problem of the present disclosure is to provide a stable pharmaceutical formulation that may be administered intraocularly.

A stable pharmaceutical formulation according to one embodiment of the present disclosure contains: (A) a recombinant fusion protein; (B) a surfactant; (C) a sugar or a derivative thereof; and (D) a buffer.

In one embodiment of the present disclosure, the pharmaceutical formulation may be in a liquid form.

In one embodiment of the present disclosure, the recombinant fusion protein (A) may comprise a vascular endothelial growth factor (VEGF) antagonist.

In one embodiment of the present disclosure, the recombinant fusion protein (A) may comprise a human VEGF receptor extracellular domain.

In one embodiment of the present disclosure, the recombinant fusion protein (A) may comprise human VEGF receptor extracellular domain 1, 2, or a mixture thereof.

In one embodiment of the present disclosure, the recombinant fusion protein (A) may comprise an immunoglobulin G (IgG) Fc region, preferably a human immunoglobulin G (IgG) Fc region.

In one embodiment of the present disclosure, the recombinant fusion protein (A) may comprise: (1) a VEGF receptor extracellular domain 1 component comprising amino acids 27 to 129 of SEQ ID NO: 2; (2) a VEGF receptor extracellular domain 2 component comprising amino acids 130 to 231 of SEQ ID NO: 2; and (3) an Fc region component comprising amino acids 232 to 457 of SEQ ID NO: 2. The amino acid sequence of SEQ ID NO: 2 may be encoded by the polynucleotide sequence of SEQ ID NO: 1.

In one embodiment of the present disclosure, the recombinant fusion protein (A) may comprise aflibercept.

In one embodiment of the present disclosure, the concentration of the recombinant fusion protein (A) may be 5 to 100 mg/ml.

In one embodiment of the present disclosure, the surfactant (B) may comprise polysorbate, poloxamer, or a mixture thereof.

In one embodiment of the present disclosure, the surfactant (B) may comprise polysorbate 20, polysorbate 40, polysorbate 60, polysorbate 80, or a mixture of two or more thereof.

In one embodiment of the present disclosure, the surfactant (B) may comprise polysorbate 20.

In one embodiment of the present disclosure, the concentration of the surfactant (B) may be 0.01 to 0.1% (w/v).

In one embodiment of the present disclosure, the sugar (C) may comprise a monosaccharide, a disaccharide, an oligosaccharide, a polysaccharide, or a mixture of two or more thereof, and the derivative of the sugar (C) may comprise a sugar alcohol, a sugar acid, or a mixture thereof.

In one embodiment of the present disclosure, the sugar or derivative thereof (C) may comprise sorbitol, mannitol, trehalose, sucrose, or a mixture of two or more thereof.

In one embodiment of the present disclosure, the sugar or derivative thereof (C) may comprise trehalose.

In one embodiment of the present disclosure, the concentration of the sugar or derivative thereof (C) may be 1 to 20% (w/v).

In one embodiment of the present disclosure, the buffer (D) may comprise an amino acid.

In one embodiment of the present disclosure, the buffer (D) may comprise a free amino acid, an amino acid salt, or a mixture thereof.

In one embodiment of the present disclosure, the buffer (D) may comprise histidine, histidine salt, or a mixture thereof.

In one embodiment of the present disclosure, the concentration of the buffer (D) may be 1 to 20 mM.

In one embodiment of the present disclosure, the stable pharmaceutical formulation of the present disclosure may be free of acetic acid, citric acid, phosphoric acid, or mixtures thereof.

In one embodiment of the present disclosure, the stable pharmaceutical formulation of the present disclosure may further contain (E) an isotonic agent.

In one embodiment of the present disclosure, the isotonic agent (E) may comprise sodium chloride (NaCl), potassium chloride (KCl), calcium chloride (CaCl), or a mixture of two or more thereof.

In one embodiment of the present disclosure, the concentration of the isotonic agent (E) may be 30 mM or less.

In one embodiment of the present disclosure, the formulation of the present disclosure may have a pH of 5.0 to 7.0.

In one embodiment of the present disclosure, the formulation of the present disclosure may not contain NaF, KBr, NaBr, Na₂SO₄, NaSCN, K₂SO₄, or mixtures thereof.

In one embodiment of the present disclosure, the formulation of the present disclosure may be free of a chelating agent.

A stable pharmaceutical formulation according to one embodiment of the present disclosure may contain: (A) 5 to 100 mg/ml of a recombinant fusion protein comprising (1) a VEGF receptor extracellular domain 1 component comprising amino acids 27 to 129 of SEQ ID NO: 2, (2) a VEGF receptor extracellular domain 2 component comprising amino acids 130 to 231 of SEQ ID NO: 2, and (3) an Fc region component comprising amino acids 232 to 457 of SEQ ID NO:2; (B) 0.01 to 0.1% (w/v) of a surfactant; (C) 1 to 20% (w/v) of a sugar or a derivative thereof; (D) 1 to 20 mM of a buffer; and (E) 30 mM or less of an isotonic agent. The amino acid sequence of SEQ ID NO: 2 may be encoded by the polynucleotide sequence of SEQ ID NO: 1.

A stable pharmaceutical formulation according to one embodiment of the present disclosure may contain: (A) a vascular endothelial growth factor (VEGF) antagonist; (B) polysorbate; (C) a sugar or a derivative thereof; and (D) histidine.

The stable pharmaceutical formulation according to one embodiment of the present disclosure may further contain (E) sodium chloride.

A stable pharmaceutical formulation according to one embodiment of the present disclosure may contain: (A) 5 to 100 mg/ml of a recombinant fusion protein comprising (1) a VEGF receptor extracellular domain 1 component comprising amino acids 27 to 129 of SEQ ID NO: 2, (2) a VEGF receptor extracellular domain 2 component comprising amino acids 130 to 231 of SEQ ID NO: 2, and (3) an Fc region component comprising amino acids 232 to 457 of SEQ ID NO:2; (B) 0.01 to 0.1% (w/v) of polysorbate; (C) 1 to 20% (w/v) of a sugar or a derivative thereof; and (D) 1 to 20 mM of histidine.

The stable pharmaceutical formulation according to one embodiment of the present disclosure may further contain (E) 30 mM or less of sodium chloride.

In one embodiment of the present disclosure, the number of sub-visible particles having a particle diameter of equal to or more than 10.00 µm to less than 400.00 µm in the stable pharmaceutical formulation may be 50 or less as measured by HIAC after 9 months of storage at 5±3°C.

In one embodiment of the present disclosure, the stable pharmaceutical formulation may show a main component content of 98% or more after 10 days of storage at 5±3°C.

In one embodiment of the present disclosure, the stable pharmaceutical formulation may show a high-molecular-weight component content of 1% or less after 10 days of storage at 5±3°C.

In one embodiment of the present disclosure, the stable pharmaceutical formulation may show a low-molecular-weight component content of 0.05% or less after 10 days of storage at 5±3°C.

In one embodiment of the present disclosure, the stable pharmaceutical formulation may show a charge variant content of 87% or more after 9 months of storage at 5±3°C.

In one embodiment of the present disclosure, the stable pharmaceutical formulation may show a VEGF binding affinity of 90% or more after 9 months of storage at 5±3°C.

In one embodiment of the present disclosure, the stable pharmaceutical formulation may be for intraocular administration, preferably intravitreal administration.

In one embodiment of the present disclosure, the stable pharmaceutical formulation may not be subjected to a reconstitution step, a dilution step, or both, before use.

A glass vial according to one embodiment of the present disclosure is filled with the stable pharmaceutical formulation.

A pre-filled syringe according to one embodiment of the present disclosure is filled with the stable pharmaceutical formulation.

### DESCRIPTION OF SPECIFIC EMBODIMENTS

### [Stable Pharmaceutical Formulation]

A stable pharmaceutical formulation according to the present disclosure contains: (A) a recombinant fusion protein; (B) a surfactant; (C) a sugar or a derivative thereof; and (D) a buffer.

As used herein, the term "free of" means that the pharmaceutical formulation does not contain the corresponding component. In addition, the term means that the pharmaceutical formulation does not substantially contain the corresponding component, that is, contains the corresponding component in an amount within a range that does not affect the activity of the fusion protein and the stability and viscosity of the pharmaceutical formulation, for example, contains the corresponding component in an amount of 0 to 1% (w/v), 0 to 1 ppm (w/v), or 0 to 1 ppb (w/v) based on the total weight of the pharmaceutical formulation. The pharmaceutical formulation may be in a liquid form, but is not limited thereto.

### (A) Recombinant Fusion Protein

The recombinant fusion protein of the present disclosure may be a vascular endothelial growth factor (VEGF) antagonist. In addition, the recombinant fusion protein of the present disclosure may comprise: (1) a VEGF receptor extracellular domain 1 component comprising amino acids 27 to 129 of SEQ ID NO: 2; (2) a VEGF receptor extracellular domain 2 component comprising amino acids 130 to 231 of SEQ ID NO: 2; and (3) an Fc region component comprising amino acids 232 to 457 of SEQ ID NO: 2. The amino acid sequence of SEQ ID NO: 2 may be encoded by the polynucleotide sequence of SEQ ID NO: 1.

The recombinant fusion protein of the present disclosure may comprise a human VEGF receptor extracellular domain, and more specifically, may comprise a human VEGF receptor extracellular domain 1, 2, or a mixture thereof, but is not limited thereto.

The recombinant fusion protein may also comprise a human immunoglobulin G (IgG) Fc region, but is not limited thereto.

The recombinant fusion protein may comprise aflibercept, but is not limited thereto.

The concentration of the recombinant fusion protein of the present disclosure may be freely adjusted within a range that does not substantially adversely affect the stability and viscosity of the stable pharmaceutical formulation according to the present disclosure. In one embodiment of the present disclosure, the concentration of the recombinant fusion protein may be 5 to 100 mg/ml. In another embodiment of the present disclosure, the concentration of the recombinant fusion protein may be 5 to 95 mg/ml. In still another embodiment of the present disclosure, the concentration of the recombinant fusion protein may be 5 to 90 mg/ml. In still another embodiment of the present disclosure, the concentration of the recombinant fusion protein may be 5 to 85 mg/ml. In still another embodiment of the present disclosure, the concentration of the recombinant fusion protein may be 5 to 80 mg/ml. In still another embodiment of the present disclosure, the concentration of the recombinant fusion protein may be 5 to 75 mg/ml. In still another embodiment of the present disclosure, the concentration of the recombinant fusion protein may be 5 to 70 mg/ml. In still another embodiment of the present disclosure, the concentration of the recombinant fusion protein may be 5 to 65 mg/ml. In still another embodiment of the present disclosure, the concentration of the recombinant fusion protein may be 5 to 60 mg/ml. In still another embodiment of the present disclosure, the concentration of the recombinant fusion protein may be 5 to 55 mg/ml. In still another embodiment of the present disclosure, the concentration of the recombinant fusion protein may be 5 to 50 mg/ml. In still another embodiment of the present disclosure, the concentration of the recombinant fusion protein may be 5 to 45 mg/ml. In still another embodiment of the present disclosure, the concentration of the recombinant fusion protein may be 5 to 40 mg/ml.

In addition, in another embodiment of the present disclosure, the concentration of the recombinant fusion protein may be 10 to 100 mg/ml. In still another embodiment of the present disclosure, the concentration of the recombinant fusion protein may be 10 to 90 mg/ml. In still another embodiment of the present disclosure, the concentration of the recombinant fusion protein may be 10 to 80 mg/ml. In still another embodiment of the present disclosure, the concentration of the recombinant fusion protein may be 10 to 70 mg/ml. In still another embodiment of the present disclosure, the concentration of the recombinant fusion protein may be 10 to 60 mg/ml. In still another embodiment of the present disclosure, the concentration of the recombinant fusion protein may be 10 to 50 mg/ml. In still another embodiment of the present disclosure, the concentration of the recombinant fusion protein may be 10 to 40 mg/ml.

Furthermore, in another embodiment of the present disclosure, the concentration of the recombinant fusion protein may be 20 to 100 mg/ml. In still another embodiment of the present disclosure, the concentration of the recombinant fusion protein may be 20 to 90 mg/ml. In still another embodiment of the present disclosure, the concentration of the recombinant fusion protein may be 20 to 80 mg/ml. In still another embodiment of the present disclosure, the concentration of the recombinant fusion protein may be 20 to 70 mg/ml. In still another embodiment of the present disclosure, the concentration of the recombinant fusion protein may be 20 to 60 mg/ml. In still another embodiment of the present disclosure, the concentration of the recombinant fusion protein may be 20 to 50 mg/ml. In still another embodiment of the present disclosure, the concentration of the recombinant fusion protein may be 20 to 40 mg/ml.

In addition, in another embodiment of the present disclosure, the concentration of the recombinant fusion protein may be 30 to 100 mg/ml. In still another embodiment of the present disclosure, the concentration of the recombinant fusion protein may be 30 to 90 mg/ml. In still another embodiment of the present disclosure, the concentration of the recombinant fusion protein may be 30 to 80 mg/ml. In still another embodiment of the present disclosure, the concentration of the recombinant fusion protein may be 30 to 70 mg/ml. In still another embodiment of the present disclosure, the concentration of the recombinant fusion protein may be 30 to 60 mg/ml. In still another embodiment of the present disclosure, the concentration of the recombinant fusion protein may be 30 to 50 mg/ml. In still another embodiment of the present disclosure, the concentration of the recombinant fusion protein may be 30 to 40 mg/ml.

### (B) Surfactant

Examples of the surfactant include, but are not limited to, polyoxyethylene sorbitan fatty acid esters (e.g., polysorbate), polyoxyethylene alkyl ethers (e.g., Brij), alkylphenyl polyoxyethylene ethers (e.g., Triton-X), polyoxyethylene-polyoxypropylene copolymers (e.g., Poloxamer, Pluronic), sodium dodecyl sulfate (SDS), poloxamers, and mixtures thereof.

In one embodiment of the present disclosure, the surfactant may comprise polysorbate, poloxamer, or a mixture thereof, and specifically, comprise polyoxyethylene sorbitan fatty acid ester (polysorbate), poloxamer, or a mixture thereof. More specifically, the surfactant may comprise polysorbate 20, polysorbate 40, polysorbate 60, polysorbate 80, or a mixture of two or more thereof, but is not limited thereto. In another embodiment of the present disclosure, the polysorbate may comprise polysorbate 20, but is not limited thereto.

In one embodiment of the present disclosure, the concentration of the surfactant may be freely adjusted within a range that does not adversely affect the stability and viscosity of the stable pharmaceutical formulation according to the present disclosure. For example, the concentration of the surfactant may be 0.001 to 5% (w/v), or 0.005 to 2% (w/v), more preferably 0.01 to 1%, 0.01 to 0.5%, 0.01 to 0.1%, or 0.01 to 0.05% (w/v), but is not limited thereto.

### (C) Sugar or Derivative of Sugar

The sugar may comprise a monosaccharide, a disaccharide, an oligosaccharide, a polysaccharide, or a mixture of two or more thereof. Examples of the monosaccharide include, but are not limited to, glucose, fructose, galactose, and the like. Examples of the disaccharide include, but are not limited to, sucrose, lactose, maltose, trehalose, and the like. Examples of the oligosaccharide include, but are not limited to, fructooligosaccharides, galactooligosaccharides, mannan oligosaccharides, and the like. Examples of the polysaccharide include, but are not limited to, starch, glycogen, cellulose, chitin, pectin, and the like.

The derivative of the sugar may comprise a sugar alcohol, a sugar acid, or a mixture thereof. Examples of the sugar alcohol include, but are not limited to, glycerol, erythritol, threitol, arabitol, xylitol, ribitol, mannitol, sorbitol, galactitol, fucitol, iditol, inositol, volemitol, isomalt, maltitol, lactitol, maltotriitol, maltotetraitol, polyglycitol, and the like. Examples of the sugar acid include, but are not limited to, aldonic acid (such as glyceric acid), ulosonic acid (such as neuramic acid), uronic acid (such as glucuronic acid), and aldaric acid (such as tartaric acid).

In one embodiment of the present disclosure, the sugar or derivative thereof may comprise sorbitol, mannitol, trehalose, sucrose, or a mixture of two or more thereof. In another embodiment of the present disclosure, the sugar or derivative thereof may comprise trehalose, but is not limited thereto.

In one embodiment of the present disclosure, the concentration of the sugar or derivative thereof may be freely adjusted within a range that does not substantially adversely affect the stability and viscosity of the pharmaceutical formulation according to the present disclosure. For example, the concentration of the sugar or derivative thereof may be 0.1 to 30% (w/v), or 0.5 to 25% (w/v), more preferably 1 to 20% (w/v). According to one embodiment of the present disclosure, the concentration of the sugar or derivative thereof may be 0.5 to 20% (w/v) or 1 to 25% (w/v). According to one embodiment of the present disclosure, the concentration of the sugar or derivative thereof may be 1 to 20% (w/v), 2 to 15% (w/v), 4 to 13% (w/v), or 8 to 12% (w/v).

### (D) Buffer

The buffer is a neutralizing substance that minimizes the change in pH caused by acid or alkali. Examples of the buffer include phosphate, acetate, succinate, gluconate, citrate, as well as glutamate and histidine that are amino acids.

In one embodiment of the present disclosure, the buffer may comprise an amino acid, and more specifically, may comprise a free amino acid, an amino acid salt, or a mixture thereof, but is not limited thereto. In one embodiment of the present disclosure, the buffer may comprise histidine, histidine salt, or a mixture thereof, but is not limited thereto.

When a histidine salt is used as the buffer, the buffer may comprise, for example, histidine chloride, histidine acetate, histidine phosphate, histidine sulfate, or the like. The use of histidine as the buffer is preferable in terms of pH control and stability, but is not limited thereto. In addition, the pharmaceutical formulation may also contain other types of acids for pH adjustment, but according to one embodiment of the present disclosure, the pharmaceutical formulation may be free of acetic acid, citric acid, phosphoric acid, or mixtures thereof, but is not limited thereto.

In one embodiment of the present disclosure, the concentration of the buffer may be freely adjusted within a range that does not substantially adversely affect the stability and viscosity of the pharmaceutical formulation according to the present disclosure. In one embodiment of the present disclosure, the concentration of the buffer may be 1 to 20 mM or 1 to 10 mM. In one embodiment of the present disclosure, the concentration of the buffer may be 2 to 20 mM, or 2 to 10 mM. In one embodiment of the present disclosure, the concentration of the buffer may be 3 to 20 mM, or 3 to 10 mM. In one embodiment of the present disclosure, the concentration of the buffer may be 4 to 20 mM, or 4 to 10 mM. In one embodiment of the present disclosure, the concentration of the buffer may be 5 to 20 mM, or 5 to 10 mM. In one embodiment of the present disclosure, the concentration of the buffer may be 6 to 20 mM, or 6 to 10 mM. In one embodiment of the present disclosure, the concentration of the buffer may be 1 to 20 mM. In one embodiment of the present disclosure, the concentration of the buffer may be 6.4 to 9.6 mM, but is not limited thereto.

### (E) Isotonic agent

In one embodiment of the present disclosure, the pharmaceutical formulation may further contain (E) an isotonic agent.

The isotonic agent may comprise sodium chloride, potassium chloride, calcium chloride, or a mixture of two or more thereof.

In one embodiment of the present disclosure, the concentration of the isotonic agent may be freely adjusted within a range that does not substantially adversely affect the stability and viscosity of the pharmaceutical formulation according to the present disclosure. In one embodiment of the present disclosure, the concentration of the isotonic agent may be 30 mM or less. In one embodiment of the present disclosure, the concentration of the isotonic agent may be 0.00001 to 30 mM, 0.0001 to 30 mM, 0.001 to 30 mM, 0.01 to 30 mM, 0.1 to 30 mM, or 1 to 30 mM. In one embodiment of the present disclosure, the concentration of the isotonic agent may be 0.00001 to 30 mM, 0.00001 to 25 mM, or 0.00001 to 20 mM. In one embodiment of the present disclosure, the concentration of the isotonic agent may be 0.0001 to 30 mM, 0.0001 to 25 mM, or 0.0001 to 20 mM. In one embodiment of the present disclosure, the concentration of the isotonic agent may be 0.001 to 30 mM, 0.001 to 25 mM, or 0.001 to 20 mM. In one embodiment of the present disclosure, the concentration of the isotonic agent may be 0.01 to 30 mM, 0.01 to 25 mM, or 0.01 to 20 mM. In one embodiment of the present disclosure, the concentration of the isotonic agent may be 0.1 to 30 mM, 0.1 to 25 mM, or 0.1 to 20 mM. In one embodiment of the present disclosure, the concentration of the isotonic agent may be 1 to 30 mM, 1 to 25 mM, or 1 to 20 mM. In one embodiment of the present disclosure, the concentration of the isotonic agent may be 2 to 30 mM, 2 to 25 mM, or 2 to 20 mM. In one embodiment of the present disclosure, the concentration of the isotonic agent may be 3 to 30 mM, 3 to 25 mM, or 3 to 20 mM. In one embodiment of the present disclosure, the concentration of the isotonic agent may be 5 to 18 mM, 6 to 17 mM, or 7.2 to 15.6 mM, but is not limited thereto.

### (F) pH

In one embodiment of the present disclosure, the pH of the stable pharmaceutical formulation may be 5.0 to 7.0, 5.0 to 6.5, 5.5 to 7.0, or 5.5 to 6.5. When the pH is within this range, the pharmaceutical formulation may exhibit excellent long-term stability and low viscosity. The pH of the pharmaceutical formulation may be adjusted using a buffer. In other words, when the pharmaceutical formulation contains a predetermined amount of a buffer, it may exhibit a pH within the above range without a separate pH-adjusting agent. In one embodiment of the present disclosure, the pharmaceutical formulation may be free of acetic acid, citric acid, phosphoric acid, or a mixture thereof. If acetic acid (acetate), citric acid (citrate), phosphoric acid, or a mixture thereof is used as a buffer, it may be difficult to show a pH within the above range. If the pharmaceutical formulation further contains an acid (e.g., hydrochloric acid) or a base (e.g., sodium hydroxide) as a separate pH-adjusting agent, the desired stability of the recombinant fusion protein may be reduced.

### (G) Other Components

In one embodiment of the present disclosure, the stable pharmaceutical formulation may be free of a preservative. Examples of the preservative include octadecyldimethylbenzyl ammonium chloride, hexamethonium chloride, benzalkonium chloride, benzethonium chloride, phenol, butyl alcohol, benzyl alcohol, alkyl parabens, catechol, resorcinol, cyclohexanol, 3-pentanol, m-cresol, and the like. When the preservative is contained, it may not help improve the stability of the pharmaceutical formulation.

In one embodiment of the present disclosure, the stable pharmaceutical formulation may be free of NaF, KBr, NaBr, Na₂SO₄, NaSCN, K₂SO₄, or a mixture thereof, and may also be free of a chelating agent, but is not limited thereto.

### (H) "Stable" Pharmaceutical Formulation

The term "stable" in the "stable" pharmaceutical formulation of the present disclosure means that the recombinant fusion protein according to the present disclosure essentially retains its physical stability and/or chemical stability and/or biological activity during production and/or storage thereof. Various analytical techniques for measuring antibody stability are readily available in the art. Stability may be measured for a selected period of time at a selected temperature.

Physical stability may be assessed by methods known in the art, which include measurement of a sample's apparent attenuation of light (absorbance, or optical density). This measurement of light attenuation is related to the turbidity of a formulation. In addition, for physical stability, the contents of high-molecular-weight components, the contents of low-molecular-weight components, the amounts of intact proteins, the number of sub-visible particles, and the like, may be measured.

Chemical stability can be assessed by, for example, detecting and quantifying chemically altered forms of the recombinant fusion protein. Chemical stability includes charge alteration (for example, occurring as a result of deamidation or oxidation) which can be evaluated by, for example, ion-exchange chromatography. For chemical stability, charge variants (acidic or basic peaks) may be measured.

Biological activity may be assessed by methods known in the art. For example, antigen binding affinity may be measured by ELISA.

In one embodiment of the present disclosure, the term "stable" pharmaceutical formulation means a pharmaceutical formulation satisfying one or more of the following criteria.

### (H)-1 Turbidity

- a pharmaceutical formulation having an absorbance A₆₀₀ of 0 to 0.0700, or 0 to 0.0400, as measured by a spectrophotometer after 4 weeks of storage at a temperature of 5±3°C;

a pharmaceutical formulation having an absorbance A₆₀₀ of 0 to 0.0700, or 0 to 0.0400, as measured by a spectrophotometer after 4 weeks of storage at a temperature of 10°C, 15°C, 20°C, 25°C, 30°C or 35°C under a closed condition;
a pharmaceutical formulation having an absorbance A₆₀₀ of 0 to 0.0700, or 0 to 0.0400, as measured by a spectrophotometer after 4 weeks of storage at a temperature of 40±2°C and a relative humidity of 75±5% under a closed condition;

### (H)-2 Content of Main Component (Main Peak)

- a pharmaceutical formulation in which the content of a main component after 9 months of storage at a temperature of 5±3°C is 98 to 99% as measured by SE-HPLC;
- a pharmaceutical formulation in which the content of a main component after 4 weeks of storage at a temperature of 40±2°C and a relative humidity of 75±5% under a closed condition is 89 to 92% as measured by SE-HPLC;

**(H)-3 Content of High-Molecular-Weight Components** (a peak whose retention time is earlier than that of the main peak (intact recombinant fusion protein))
- a pharmaceutical formulation in which the content of high-molecular-weight components after 9 months of storage at a temperature of 5±3°C is 98 to 100% as measured by SE-HPLC;
a pharmaceutical formulation in which the content of high-molecular-weight components after 4 weeks of storage at a temperature of 40±2°C and a relative humidity of 75±5% under a closed condition is 7 to 10% as measured by SE-HPLC;

**(H)-4 Content of Low-Molecular-Weight Components** (a peak whose retention time is later than that of the main peak (intact recombinant fusion protein))
a pharmaceutical formulation in which the content of low-molecular-weight components after 9 months of storage at a temperature of 5±3°C is 0.0 to 0.1% as measured by SE-HPLC;
a pharmaceutical formulation in which the content of low-molecular-weight components after 4 weeks of storage at a temperature of 40±2°C and a relative humidity of 75±5% under a closed condition is 0.0 to 1.2% as measured by SE-HPLC;

### (H)-5 Content of one intact recombinant human VEGF receptor extracellular domain 1, one recombinant human VEGF receptor extracellular domain 2 and recombinant human IgG Fc region (1VEGF1 + 1VEGF2 + Fc)

- a pharmaceutical formulation in which the content of one intact recombinant human VEGF receptor extracellular domain 1, one recombinant human VEGF receptor extracellular domain 2 and a recombinant human IgG Fc region (1VEGF1 + 1VEGF2 + Fc) after 9 months of storage at a temperature of 5±3°C is 96% to 98% as measured by reduced CE-SDS;
- a pharmaceutical formulation in which the content of one intact recombinant human VEGF receptor extracellular domain 1, one recombinant human VEGF receptor extracellular domain 2 and a recombinant human IgG Fc region (1VEGF1 + 1VEGF2 + Fc) after 4 weeks of storage at a temperature of 40±2°C and a relative humidity of 75±5% under a closed condition is 90% to 96% as measured by reduced CE-SDS;

### (H)-6 Number of Sub-Visible Particles

- a pharmaceutical formulation in which the number of sub-visible particles (≥2.00 µm, <400.00 µm) after 9 months of storage at a temperature of 5±3°C is 0 to 100 as measured by HIAC;
- a pharmaceutical formulation in which the number of sub-visible particles (≥10.00 µm, <400.00 µm) after 9 months of storage at a temperature of 5±3°C is 0 to 50 as measured by HIAC;
- a pharmaceutical formulation in which the number of sub-visible particles (≥25.00 µm, <400.00 µm) after 9 months of storage at a temperature of 5±3°C is 0 to 5 as measured by HIAC;
- a pharmaceutical formulation in which the number of sub-visible particles (≥2.00 µm, <400.00 µm) after 4 weeks of storage at a temperature of 40±2°C and a relative humidity of 75±5% under a closed condition is 0 to 100 as measured by HIAC;
- a pharmaceutical formulation in which the number of sub-visible particles (≥10.00 µm, <400.00 µm) after 4 weeks of storage at a temperature of 40±2°C and a relative humidity of 75±5% under a closed condition is 0 to 50 as measured by HIAC;
   a pharmaceutical formulation in which the number of sub-visible particles (≥25.00 µm, <400.00 µm) after 4 weeks of storage at a temperature of 40±2°C and a relative humidity of 75±5% under a closed condition is 0 to 5 as measured by HIAC;
- a pharmaceutical formulation in which the number of sub-visible particles (≥1.00 µm, <400.00 µm) after 9 months of storage at a temperature of 5±3°C is 0 to 5,000 as measured by MFI;
- a pharmaceutical formulation in which the number of sub-visible particles (≥10.00 µm, <400.00 µm) after 9 months of storage at a temperature of 5±3°C is 0 to 100 as measured by MFI;
- a pharmaceutical formulation in which the number of sub-visible particles (≥25.00 µm, <400.00 µm) after 9 months of storage at a temperature of 5±3°C is 0 to 15 as measured by MFI;
- a pharmaceutical formulation in which the number of sub-visible particles (≥10.0 µm, <100.00 µm) after 4 weeks of storage at a temperature of 5±3°C is 0 to 1,000 as measured by MFI;
- a pharmaceutical formulation in which the number of sub-visible particles (≥10.0 µm, <100.00 µm) after 4 weeks of storage at a temperature of 5±3°C is 0 to 100 as measured by MFI;
- a pharmaceutical formulation in which the number of sub-visible particles (≥25.0 µm, <100.00 µm) after 4 weeks of storage at a temperature of 5±3°C is 0 to 100 as measured by MFI;
- a pharmaceutical formulation in which the number of sub-visible particles (≥25.0 µm, <100.00 µm) after 4 weeks of storage at a temperature of 5±3°C is 0 to 10 as measured by MFI;
- a pharmaceutical formulation in which the number of sub-visible particles (≥1.00 µm, <100.00 µm) after 4 weeks of storage at a temperature of 40±2°C is 0 to 5,000 as measured by MFI;
- a pharmaceutical formulation in which the number of sub-visible particles (≥1.00 µm, <100.00 µm) after 4 weeks of storage at a temperature of 40±2°C is 0 to 2,500 as measured by MFI;
- a pharmaceutical formulation in which the number of sub-visible particles (≥10.00 µm, <100.00 µm) after 4 weeks of storage at a temperature of 40±2°C is 0 to 1,000 as measured by MFI;
- a pharmaceutical formulation in which the number of sub-visible particles (≥10.00 µm, <100.00 µm) after 4 weeks of storage at a temperature of 40±2°C is 0 to 100 as measured by MFI;
- a pharmaceutical formulation in which the number of sub-visible particles (≥25.00 µm, <100.00 µm) after 4 weeks of storage at a temperature of 40±2°C is 0 to 100 as measured by MFI;
- a pharmaceutical formulation in which the number of sub-visible particles (≥25.00 µm, <100.00 µm) after 4 weeks of storage at a temperature of 40±2°C is 0 to 10 as measured by MFI;
- a pharmaceutical formulation in which the number of sub-visible particles (≥1.00 µm, <400.00 µm) after 4 weeks of storage at a temperature of 40±2°C at a relative humidity of 75±5% under a closed condition is 0 to 20,000 as measured by MFI;
- a pharmaceutical formulation in which the number of sub-visible particles (≥10.00 µm, <400.00 µm) after 4 weeks of storage at a temperature of 40±2°C at a relative humidity of 75±5% under a closed condition is 0 to 70 as measured by MFI;
- a pharmaceutical formulation in which the number of sub-visible particles (≥25.00 µm, <400.00 µm) after 4 weeks of storage at a temperature of 40±2°C at a relative humidity of 75±5% under a closed condition is 0 to 15 as measured by MFI;

### (H)-7 Oxidation Rate

- a pharmaceutical formulation in which the oxidation rate of heavy-chain Met 192 after 4 weeks of storage at a temperature of 5±3°C is 0% to 7% as measured by LC-MS;
- a pharmaceutical formulation in which the oxidation rate of heavy-chain Met 192 after 4 weeks of storage at a temperature of 40±2°C and a relative humidity of 75±5% under a closed condition is 0% to 6% as measured by LC-MS;

### (H)-8 Charge Variants

- a pharmaceutical formulation in which the relative content of theoretical peak 4 to peak 11 is 89% to 91% as measured by cIEF after 4 weeks of storage at a temperature of 5±3°C;
- a pharmaceutical formulation in which the sum of theoretical peak 4 to peak 11 is 86% to 88% as measured by cIEF after 9 months of storage at a temperature of 5±3°C;
- a pharmaceutical formulation in which the sum of theoretical peak 4 to peak 11 is 86% to 88% as measured by cIEF after 4 weeks of storage at a temperature of 40±2°C and a relative humidity of 75±5% under a closed condition;
- a pharmaceutical formulation in which the relative content of theoretical peak 4 to peak 11 is 86% to 88% as measured by cIEF after 4 weeks of storage at a temperature of 40±2°C and a relative humidity of 75±5% under a closed condition;

### (H)-9 VEGF Binding Affinity

- a pharmaceutical formulation having a VEGF binding affinity of 75% to 95% as measured by ELISA after 9 months of storage at a temperature of 5±3°C; and
- a pharmaceutical formulation having a VEGF binding affinity of 60% to 75% as measured by ELISA after 4 weeks of storage at a temperature of 40±2°C and a relative humidity of 75±5% under a closed condition.

### [Method for Preparing Stable Pharmaceutical Formulation]

The stable pharmaceutical formulation of the present disclosure may be prepared using any known method which is not limited to a particular method. For example, the stable pharmaceutical formulation may be prepared by adding a buffer to a solution containing a surfactant, a sugar or a derivative thereof and an isotonic agent while adjusting the pH of the solution, and then adding a recombinant fusion protein to the mixed solution. Alternatively, the pharmaceutical formulation may be prepared by preparing a solution containing some excipients in the final step of a purification process, and then adding the remaining component to the solution. For example, the pharmaceutical formulation may be prepared by preparing a solution containing a recombinant fusion protein, a buffer, a sugar or a derivative thereof and an isotonic agent in the final step of a purification process, and then adding a surfactant to the solution.

In addition, the method for preparing the formulation may not comprise a freeze-drying step.

When the preparation method does not comprise the freeze-drying step, for example, the pharmaceutical formulation prepared according to the present disclosure may be treated by sterilization, and then immediately placed in a sealed container, such as a glass vial or pre-filled syringe, which is a primary packaging material.

### [Method of Use of Stable Pharmaceutical Formulation]

The stable pharmaceutical formulation according to the present disclosure may be used to treat diseases caused by release of VEGF. Examples of diseases caused by release of VEGF include, but are not limited to, neovascular (wet) age-related macular degeneration, visual impairment due to retinal vein occlusion (central retinal vein occlusion or branch retinal vein occlusion) macular edema, visual impairment due to diabetic macular edema, and visual impairment due to choroidal neovascularization caused by pathologic myopia.

The stable pharmaceutical formulation according to the present disclosure may be used in a single-dosage form or a multiple-dosage form, and should be administered by a qualified physician with experience in intraocular administration (injection).

The concentrations of other components, including the recombinant fusion protein, in the pharmaceutical formulation, are as described above, and the total volume of the pharmaceutical formulation may be 0.05 to 3 mL.

The dosage and timing of administration of the pharmaceutical formulation may vary depending on the kind of disease, the severity and course of the disease, the patient's health and response to treatment, and the judgment of the treating physician, and the recommended dosage thereof is limited to 2 mg (equivalent to about 50 microliters), but intervals and timing of administration thereof are not limited. For example, a single product comprising the pharmaceutical formulation may be administered into one eye at a dose of 2 mg (as the concentration of the recombinant fusion protein), and then the same dose may be administered at intervals of 2 weeks to 2 months. Dosage intervals longer than 16 weeks have not been studied.

In one embodiment of the present disclosure, the stable pharmaceutical formulation may not be subjected to a reconstitution step, a dilution step, or both, before use.

### [Treatment Method and Stabilization Method]

The present disclosure also provides a method for treating a patient, who has a disease caused by VEGF release, by using a stable pharmaceutical formulation containing: (A) a recombinant fusion protein; (B) a surfactant; (C) a sugar or a derivative thereof; and (D) a buffer.

The present disclosure also provides a method of stabilizing a stable pharmaceutical formulation containing: (A) a recombinant fusion protein; (B) a surfactant; (C) a sugar or a derivative thereof; and (D) a buffer, by providing pharmaceutical formulation conditions for stabilizing the recombinant fusion protein.

In one embodiment of the treating method or the stabilizing method, the pharmaceutical formulation may be in a liquid form.

In one embodiment of the treating method or the stabilizing method, the recombinant fusion protein (A) may comprise a vascular endothelial growth factor (VEGF) antagonist.

In one embodiment of the treating method or the stabilizing method, the recombinant fusion protein (A) may comprise a human VEGF receptor extracellular domain.

In one embodiment of the treating method or the stabilizing method, the recombinant fusion protein (A) may comprise human VEGF receptor extracellular domain 1, 2, or a mixture thereof.

In one embodiment of the treating method or the stabilizing method, the recombinant fusion protein (A) may comprise an immunoglobulin G (IgG) Fc region, preferably a human immunoglobulin G (IgG) Fc region.

In one embodiment of the treating method or the stabilizing method, the recombinant fusion protein (A) may comprise: (1) a VEGF receptor extracellular domain 1 component comprising amino acids 27 to 129 of SEQ ID NO: 2; (2) a VEGF receptor extracellular domain 2 component comprising amino acids 130 to 231 of SEQ ID NO: 2; and (3) an Fc region component comprising amino acids 232 to 457 of SEQ ID NO: 2.

In one embodiment of the treating method or the stabilizing method, the recombinant fusion protein (A) may comprise aflibercept.

In one embodiment of the treating method or the stabilizing method, the concentration of the recombinant fusion protein (A) may be 5 to 100 mg/ml.

In one embodiment of the treating method or the stabilizing method, the surfactant (B) may comprise polysorbate, poloxamer, or a mixture thereof.

In one embodiment of the treating method or the stabilizing method, the surfactant (B) may comprise polysorbate 20, polysorbate 40, polysorbate 60, polysorbate 80, or a mixture of two or more thereof.

In one embodiment of the treating method or the stabilizing method, the surfactant (B) may comprise polysorbate 20.

In one embodiment of the treating method or the stabilizing method, the concentration of the surfactant (B) may be 0.01 to 0.1% (w/v).

In one embodiment of the treating method or the stabilizing method, the sugar (C) may comprise a monosaccharide, a disaccharide, an oligosaccharide, a polysaccharide, or a mixture of two or more thereof, and the derivative of the sugar (C) may comprise a sugar alcohol, a sugar acid, or a mixture thereof.

In one embodiment of the treating method or the stabilizing method, the sugar or derivative thereof (C) may comprise sorbitol, mannitol, trehalose, sucrose, or a mixture of two or more thereof.

In one embodiment of the treating method or the stabilizing method, the sugar or derivative thereof (C) may comprise trehalose.

In one embodiment of the treating method or the stabilizing method, the concentration of the sugar or derivative thereof (C) may be 1 to 20% (w/v).

In one embodiment of the treating method or the stabilizing method, the buffer (D) may comprise an amino acid.

In one embodiment of the treating method or the stabilizing method, the buffer (D) may comprise a free amino acid, an amino acid salt, or a mixture thereof.

In one embodiment of the treating method or the stabilizing method, the buffer (D) may comprise histidine, histidine salt, or a mixture thereof.

In one embodiment of the treating method or the stabilizing method, the concentration of the buffer (D) may be 1 to 20 mM.

In one embodiment of the treating method or the stabilizing method, the stable pharmaceutical formulation of the present disclosure may be free of acetic acid, citric acid, phosphoric acid, or a mixture thereof.

In one embodiment of the treating method or the stabilizing method, the stable pharmaceutical formulation of the present disclosure may further contain (E) an isotonic agent.

The isotonic agent (E) may comprise sodium chloride (NaCl), potassium chloride (KCl), calcium chloride (CaCl), or a mixture of two or more thereof.

In one embodiment of the treating method or the stabilizing method, the content of the isotonic agent (E) may be 30 mM or less.

In one embodiment of the treating method or the stabilizing method, the formulation of the present disclosure may have a pH of 5.0 to 7.0.

In one embodiment of the treating method or the stabilizing method, the formulation of the present disclosure may be free of NaF, KBr, NaBr, Na₂SO₄, NaSCN, K₂SO₄, or a mixture thereof.

In one embodiment of the present disclosure, the formulation of the present disclosure may be free of a chelating agent.

The stable pharmaceutical formulation according to one embodiment of the treating method or the stabilizing method may contain: (A) 5 to 100 mg/ml of a recombinant fusion protein comprising (1) a VEGF receptor extracellular domain 1 component comprising amino acids 27 to 129 of SEQ ID NO: 2, (2) a VEGF receptor extracellular domain 2 component comprising amino acids 130 to 231 of SEQ ID NO: 2, and (3) an Fc region component comprising amino acids 232 to 457 of SEQ ID NO:2; (B) 0.01 to 0.1% (w/v) of a surfactant; (C) 1 to 20% (w/v) of a sugar or a derivative thereof; and (D) 1 to 20 mM of a buffer. The pharmaceutical formulation according to one embodiment of the treating method or the stabilizing method may further contain (E) 30 mM or less of an isotonic agent.

The stable pharmaceutical formulation according to one embodiment of the treating method or the stabilizing method may contain: (A) a vascular endothelial growth factor (VEGF) antagonist; (B) polysorbate; (C) a sugar or a derivative thereof; and (D) histidine. The stable pharmaceutical formulation according to one embodiment of the treating method or the stabilizing method may further contain (E) sodium chloride.

The stable pharmaceutical formulation according to one embodiment of the treating method or the stabilizing method may contain: (A) 5 to 100 mg/ml of a recombinant fusion protein comprising (1) a VEGF receptor extracellular domain 1 component comprising amino acids 27 to 129 of SEQ ID NO: 2, (2) a VEGF receptor extracellular domain 2 component comprising amino acids 130 to 231 of SEQ ID NO: 2, and (3) an Fc region component comprising amino acids 232 to 457 of SEQ ID NO:2; (B) 0.01 to 0.1% (w/v) of polysorbate; (C) 1 to 20% (w/v) of a sugar or a derivative thereof; and (D) 1 to 20 mM of histidine. The stable pharmaceutical formulation according to one embodiment of the treating method or the stabilizing method may further contain (E) 30 mM or less of sodium chloride.

The number of sub-visible particles having a particle diameter of equal to or more than 10.00 µm to less than 400.00 µm in the stable pharmaceutical formulation according to one embodiment of the treating method or the stabilizing method may be 50 or less as measured by HIAC after 9 months of storage at 5±3°C.

The stable pharmaceutical formulation according to one embodiment of the treating method or the stabilizing method may show a main component content of 98% or more after 10 days of storage at 5±3°C.

The stable pharmaceutical formulation according to one embodiment of the treating method or the stabilizing method may show a high-molecular-weight component content of 1% or less after 10 days of storage at 5±3°C.

The stable pharmaceutical formulation according to one embodiment of the treating method or the stabilizing method may show a low-molecular-weight component content of 0.05% or less after 10 days of storage at 5±3°C.

The stable pharmaceutical formulation according to one embodiment of the treating method or the stabilizing method may show a charge variant content of 87% or more after 9 months of storage at 5±3°C.

The stable pharmaceutical formulation according to one embodiment of the treating method or the stabilizing method may show a VEGF binding affinity of 90% or more after 9 months of storage at 5±3°C.

In one embodiment of the treating method or the stabilizing method, the stable pharmaceutical formulation may be for intraocular administration, preferably intravitreal administration.

In one embodiment of the treating method or the stabilizing method, the stable pharmaceutical formulation may not be subjected to a reconstitution step, a dilution step, or both, before use.

A glass vial according to one embodiment of the treating method or the stabilizing method may be filled with the stable pharmaceutical formulation.

A pre-filled syringe according to one embodiment of the treating method or the stabilizing method may be filled with the stable pharmaceutical formulation.

### [Product]

The present disclosure also provides a product comprising: the stable pharmaceutical formulation; and a container receiving the stable pharmaceutical formulation in a sealed state.

The stable pharmaceutical formulation is as described above.

In one embodiment of the present disclosure, the container may be formed of a material such as glass, a polymer (plastic), a metal or the like, but is not limited thereto. In one embodiment of the present disclosure, the container is a bottle, a vial, a cartridge, a syringe (pre-filled syringe), or a tube, but is not limited thereto. In one embodiment of the present disclosure, the container may be a glass or polymer vial, or a glass or polymer pre-filled syringe.

Specific product forms of the above-described vial, cartridge, pre-filled syringe, and methods of filling the stabile pharmaceutical formulation into the vial, cartridge, pre-filled syringe, may be readily available or implemented by any person skilled in the technical field to which the present disclosure pertains. For example, U.S. Pat. Nos. 4,861,335 and 6,331,174, etc., disclose the specific product form of a pre-filled syringe and a filling method. The above-described vial, cartridge, pre-filled syringe may be a commercially available product or may be a product separately manufactured considering the physical properties of the stable pharmaceutical formulation, an area to which the formulation is to be administered, the dose of the formulation, and the like.

In one embodiment of the present disclosure, the product may further comprise instructions providing a method of using the stable pharmaceutical formulation, a method of storing the formulation, or both. The method of using the formulation includes a method for treating a disease caused by VEGF release, and may include the route of administration, the dose of the formulation, and the timing of administration.

In one embodiment of the present disclosure, the product may comprise other required utensils (e.g., a needle, a syringe, etc.) from a commercial viewpoint and a user viewpoint.

Hereinafter, the present disclosure will be described in detail with reference to examples. However, the following examples serve merely to illustrate the present disclosure, and the scope of the present disclosure is not limited to the following examples.

### Examples

The recombinant fusion protein used in the following experimental examples was aflibercept incubated and purified at the Celltrion Research Institute.

The physical stability, chemical stability and biological activity of pharmaceutical formulations used in the following experimental examples were measured using the following methods.

### - Turbidity

The absorbance at 600 nm was measured using a UV-Vis spectrophotometer.

### - Content of Main Component

The main component content (main peak %) was measured using size exclusion high-performance liquid chromatography (SE-HPLC).

### - Content of High-Molecular-Weight Components

The content of high-molecular-weight components (pre-peak %) was measured using size exclusion high-performance liquid chromatography (SE-HPLC).

### - Content of Low-Molecular-Weight Components

The content of low-molecular-weight components (post-peak %) was measured using size exclusion high-performance liquid chromatography (SE-HPLC).

### - Content of one recombinant human VEGF receptor extracellular domain 1, one recombinant human VEGF receptor extracellular domain 2 and recombinant human IgG Fc region (1VEGF1 + 1VEGF2 + Fc)

The content (%) of one intact recombinant human VEGF receptor extracellular domain 1, one recombinant human VEGF receptor extracellular domain 2 and a recombinant human IgG Fc region (1VEGF1 + 1VEGF2 + Fc) was measured using reduced capillary electrophoresis-sodium dodecyl sulfate (R CE-SDS).

### - Number of Sub-Visible Particles

The number of sub-visible particles was measured using Micro Flow Imaging (MFI) and a light-shielding particle counter (model: HIAC 9703).

### - Oxidation

The oxidation (%) of heavy-chain Met 192 was measured by peptide mapping using liquid chromatography-mass spectrometry (LC-MS).

### - Charge Variants (relative content of peak 4 to peak 11 in a total of 12 peaks)

Acidic and basic peaks (%) were measured by Capillary Iso-Electric Focusing (cIEF).

### - VEGF Binding Affinity

VEGF binding affinity (%) was measured by Enzyme-Linked ImmunoSorbent Assay (ELISA).

### Experimental Example 1: Comparison at Different Concentrations of Histidine Buffer; Comparison at pH 5.5 and pH 6.5; Comparison at Different Concentrations of Sodium Chloride Isotonic Agent; Comparison at Different Concentrations of Trehalose; and Confirmation of Superiority of the Formulation of the Present Disclosure

For preparation of pharmaceutical formulations to be used in Experimental Example 1, each buffer was prepared so as to have a predetermined pH, and trehalose and sodium chloride was added thereto. Then, a recombinant fusion protein was added thereto and a surfactant was added, thereby preparing the samples shown in Table 1 below. The specific content of each component is shown in Table 1 below. Examples 1 to 9 and Comparative Example 1 were samples to which aflibercept purified at the Celltrion Research Institute was added, and Comparative Example 2 was a sample prepared by adding Regeneron's Eylea (aflibercept) to the same pharmaceutical formulation as that of Comparative Example 1. The target volume of each sample for filling a glass vial was 0.278 ml.

The pharmaceutical formulations prepared according to Examples 1 to 9 and Comparative Examples 1 and 2 were stored at a temperature of 5±3°C and at a temperature of 40±2°C and a relative humidity of 75±5%, and measured for their stabilities after 0 week, 2 weeks and 4 weeks at a temperature of 5±3°C, and for their stabilities after 2 weeks and 4 weeks at a temperature of 40±2°C and a relative humidity of 75±5%. The results of the measurement are shown in Tables 2 to 15 below.

**[Table 1] Constitution of Experimental Example 1**

| | **Buffer** | **pH** | **Sugar** | **Isotonic agent** | **Surfactant** | **Protein concentration** |
|---|---|---|---|---|---|---|
| **Example 1** | 8.0 mM histidine | 5.5 | 10 % (w/v) trehalose | 13.0 mM sodium chloride | 0.03% (w/v) polysorbate 20 | 40 mg/mL |
| **Example 2** | 8.0 mM histidine | 6.0 | 10 % (w/v) trehalose | 13.0 mM sodium chloride | 0.03% (w/v) polysorbate 20 | 40 mg/mL |
| **Example 3** | 8.0 mM histidine | 6.5 | 10% (w/v) trehalose | 13.0 mM sodium chloride | 0.03% (w/v) polysorbate 20 | 40 mg/mL |
| **Example 4** | 6.4 mM histidine | 6.0 | 10% (w/v) trehalose | 13.0 mM sodium chloride | 0.03% (w/v) polysorbate 20 | 40 mg/mL |
| **Example 5** | 9.6 mM histidine | 6.0 | 10% (w/v) trehalose | 13.0 mM sodium chloride | 0.03% (w/v) polysorbate 20 | 40 mg/mL |
| **Example 6** | 8.0 mM histidine | 6.0 | 10% (w/v) trehalose | 10.4 mM sodium chloride | 0.03% (w/v) polysorbate 20 | 40 mg/mL |
| **Example 7** | 8.0 mM histidine | 6.0 | 10% (w/v) trehalose | 15.6 mM sodium chloride | 0.03% (w/v) polysorbate 20 | 40 mg/mL |
| **Example 8** | 8.0 mM histidine | 6.0 | 8% (w/v) trehalose | 13.0 mM sodium chloride | 0.03% (w/v) polysorbate 20 | 40 mg/mL |
| **Example 9** | 8.0 mM histidine | 6.0 | 12% (w/v) trehalose | 13.0 mM sodium chloride | 0.03% (w/v) polysorbate 20 | 40 mg/mL |
| **Comparative Example 1** | 10 mM sodium phosphate | 6.2 | 5% (w/v) sucrose | 40.0 mM sodium chloride | 0.03% (w/v) polysorbate 20 | 40 mg/mL |
| **Comparative Example 2** | 10 mM sodium phosphate | 6.2 | 5% (w/v) sucrose | 40.0 mM sodium chloride | 0.03% (w/v) polysorbate 20 | 40 mg/mL |

**[Table 2] Turbidity**

| | **After 0 week at 5±3°C** | **After 2 weeks at 5±3°C** | **After 4 weeks at 5±3°C** | **After 2 weeks at 40±2°C and 75±5%** | **After 4 weeks at 40±2°C and 75±5%** |
|---|---|---|---|---|---|
| **Example 1** | 0.0154 | 0.0163 | 0.0275 | 0.0181 | 0.0143 |
| **Example 2** | 0.0230 | 0.0196 | 0.0252 | 0.0188 | 0.0258 |
| **Example 3** | 0.0214 | 0.0214 | 0.0207 | 0.0212 | 0.0203 |
| **Example 4** | 0.0200 | 0.0192 | 0.0164 | 0.0283 | 0.0350 |
| **Example 5** | 0.0156 | 0.0172 | 0.0206 | 0.0365 | 0.0245 |
| **Example 6** | 0.0150 | 0.0163 | 0.0240 | 0.0153 | 0.0190 |
| **Example 7** | 0.0124 | 0.0165 | 0.0162 | 0.0229 | 0.0191 |
| **Example 8** | 0.0157 | 0.0259 | 0.0176 | 0.0194 | 0.0210 |
| **Example 9** | 0.0126 | 0.0212 | 0.0394 | 0.0153 | 0.0379 |
| **Comparative Example 1** | 0.0203 | 0.0197 | 0.0207 | 0.0240 | 0.0191 |
| **Comparative Example 2** | 0.0105 | 0.0178 | 0.0170 | 0.0197 | 0.0181 |

Referring to Table 2 above, it can be seen that the formulations of Examples 1 to 9 satisfying the ranges specified in the present disclosure showed very low turbidities even after 4 weeks at a temperature of 5±3°C, suggesting that they were transparent pharmaceutical formulations. In particular, it can be seen that these formulations showed an absorbance of 0.0400 or lower even after 4 weeks at a temperature of 40±2°C and a relative humidity of 75±5%. It can be seen that these results are similar to those obtained for the conventional formulations (Comparative Examples 1 and 2).

**[Table 3] Intact main component content (main peak %)**

| | **After 0 week at 5±3°C** | **After 2 weeks at 5±3°C** | **After 4 weeks at 5±3°C** | **After 2 weeks at 40±2°C and 75±5%** | **After 4 weeks at 40±2°C and 75±5%** |
|---|---|---|---|---|---|
| **Example 1** | 99.33 | 99.32 | 99.18 | 94.52 | 89.41 |
| **Example 2** | 99.04 | 98.96 | 98.84 | 94.85 | 91.13 |
| **Example 3** | 98.89 | 98.76 | 98.61 | 94.96 | 91.47 |
| **Example 4** | 98.98 | 98.89 | 98.88 | 95.11 | 91.31 |
| **Example 5** | 99.08 | 98.94 | 98.95 | 95.11 | 91.27 |
| **Example 6** | 99.14 | 99.04 | 98.86 | 95.17 | 91.89 |
| **Example 7** | 98.91 | 98.92 | 98.95 | 95.20 | 91.27 |
| **Example 8** | 99.06 | 99.03 | 98.88 | 94.49 | 89.88 |
| **Example 9** | 99.01 | 99.04 | 98.94 | 95.63 | 92.22 |
| **Comparative Example 1** | 98.90 | 98.61 | 98.79 | 92.00 | 8607 |
| **Comparative Example 2** | 98.09 | 97.95 | 97.98 | 92.97 | 89.34 |

Referring to Table 3 above, it can be seen that, after 4 weeks at a temperature of 5±3°C, the main component contents of the pharmaceutical formulations of all Examples 1 to 9 satisfying the ranges specified in the present disclosure were similar to those of Comparative Examples 1 and 2. However, it can be seen that, after 4 weeks at a temperature of 40±2°C and a relative humidity of 75±5%, the main component contents of the pharmaceutical formulations of Examples 1 to 9 were higher than those of Comparative Examples 1 and 2, suggesting that the formulation of the present disclosure is an improved pharmaceutical formulation capable of more stably storing a desired recombinant fusion protein.

**[Table 4] Content (%) of high-molecular-weight components**

| | **After 0 week at 5±3°C** | **After 2 weeks at 5±3°C** | **After 4 weeks at 5±3°C** | **After 2 weeks at 40±2°C and 75±5%** | **After 4 weeks at 40±2°C and 75±5%** |
|---|---|---|---|---|---|
| **Example 1** | 0.66 | 0.68 | 0.82 | 4.70 | 9.44 |
| **Example 2** | 0.96 | 1.03 | 1.15 | 4.69 | 8.24 |
| **Example 3** | 1.10 | 1.23 | 1.35 | 4.97 | 8.43 |
| **Example 4** | 1.02 | 1.08 | 1.10 | 4.47 | 8.10 |
| **Example 5** | 0.90 | 1.06 | 1.01 | 4.41 | 8.12 |
| **Example 6** | 0.86 | 0.94 | 1.13 | 4.35 | 7.56 |
| **Example 7** | 1.07 | 1.06 | 1.04 | 4.36 | 8.12 |
| **Example 8** | 0.88 | 0.96 | 1.10 | 5.07 | 9.47 |
| **Example 9** | 0.98 | 0.94 | 0.93 | 3.94 | 7.15 |
| **Comparative Example 1** | 1.10 | 1.38 | 1.19 | 7.45 | 13.29 |
| **Comparative Example 2** | 1.89 | 2.01 | 1.99 | 6.94 | 10.53 |

Referring to Table 4 above, it can be seen that, after 4 weeks at a temperature of 5±3°C, the high-molecular-weight component contents of the pharmaceutical formulations of all Examples 1 to 9 satisfying the ranges specified in the present disclosure were similar to those of Comparative Examples 1 and 2. However, it can be seen that, after 4 weeks at a temperature of 40±2°C and a relative humidity of 75±5%, the high-molecular-weight component contents of the pharmaceutical formulations of Examples 1 to 9 were lower than those of Comparative Examples 1 and 2, suggesting that the formulation of the present disclosure is an improved pharmaceutical formulation capable of more stably storing a desired recombinant fusion protein.

**[Table 5] Content (%) of low-molecular-weight components**

| | **After 0 week at 5±3°C** | **After 2 weeks at 5±3°C** | **After 4 weeks at 5±3°C** | **After 2 weeks at 40±2°C and 75±5%** | **After 4 weeks at 40±2°C and 75±5%** |
|---|---|---|---|---|---|
| **Example 1** | 0.02 | 0.00 | 0.01 | 0.77 | 1.15 |
| **Example 2** | 0.00 | 0.01 | 0.01 | 0.47 | 0.64 |
| **Example 3** | 0.01 | 0.01 | 0.04 | 0.08 | 0.10 |
| **Example 4** | 0.00 | 0.03 | 0.02 | 0.42 | 0.59 |
| **Example 5** | 0.02 | 0.00 | 0.03 | 0.48 | 0.62 |
| **Example 6** | 0.00 | 0.01 | 0.00 | 0.48 | 0.55 |
| **Example 7** | 0.01 | 0.02 | 0.01 | 0.43 | 0.61 |
| **Example 8** | 0.06 | 0.02 | 0.02 | 0.44 | 0.65 |
| **Example 9** | 0.01 | 0.01 | 0.13 | 0.43 | 0.63 |
| **Comparative Example 1** | 0.00 | 0.01 | 0.02 | 0.54 | 0.64 |
| **Comparative Example 2** | 0.02 | 0.04 | 0.03 | 0.09 | 0.13 |

Referring to Table 5 above, it can be seen that, after 4 weeks at a temperature of 5±3°C, the low-molecular-weight component contents of the pharmaceutical formulations of all Examples 1 to 9 satisfying the ranges specified in the present disclosure were similar to those of Comparative Examples 1 and 2. In addition, it can be seen that, after 4 weeks at a temperature of 40±2°C and a relative humidity of 75±5%, the low-molecular-weight component contents of the pharmaceutical formulations of Examples 2 to 9 were similar to that of Comparative Example 1. However, it was confirmed that, at pH 5.5, the content of low-molecular-weight components in Example 1 was somewhat disadvantageous compared to those in Comparative Examples 1 to 2, and thus it was necessary to adjust the target pH and pH range to slightly increased values. The experimental results of adjusting the pH range are included in Experimental Example 2.

**[Table 6] Content (%) of one recombinant human VEGF receptor extracellular domain 1, one recombinant human VEGF receptor extracellular domain 2 and recombinant human IgG Fc region (1VEGF1 + 1VEGF2 + Fc)**

| | **After 0 week at 5±3°C** | **After 2 weeks at 5±3°C** | **After 4 weeks at 5±3°C** | **After 2 weeks at 40±2°C and 75±5%** | **After 4 weeks at 40±2°C and 75±5%** |
|---|---|---|---|---|---|
| **Example 1** | 95.43 | 95.97 | 98.30 | 95.18 | 90.60 |
| **Example 2** | 97.33 | 97.65 | 98.30 | 95.23 | 93.80 |
| **Example 3** | 97.57 | 98.04 | 98.30 | 97.32 | 96.20 |
| **Example 4** | 97.53 | 97.45 | 98.30 | 95.98 | 94.00 |
| **Example 5** | 97.45 | 97.83 | 98.30 | 95.69 | 93.80 |
| **Example 6** | 97.41 | 97.27 | 98.30 | 95.67 | 94.10 |
| **Example 7** | 96.65 | 98.35 | 97.70 | 95.74 | 93.80 |
| **Example 8** | 97.58 | 97.43 | 98.20 | 96.42 | 93.80 |
| **Example 9** | 97.52 | 96.33 | 97.90 | 95.71 | 93.90 |
| **Comparative Example 1** | 97.57 | 97.27 | 98.40 | 96.35 | 93.60 |
| **Comparative Example 2** | 97.89 | 97.15 | 98.30 | 96.74 | 96.40 |

Referring to Table 6 above, it can be seen that, after 4 weeks at a temperature of 5±3°C, the contents of one recombinant human VEGF receptor extracellular domain 1, one recombinant human VEGF receptor extracellular domain 2 and a recombinant human IgG Fc region (1VEGF1 + 1VEGF2 + Fc) in the pharmaceutical formulations of all Examples 1 to 9 satisfying the ranges specified in the present disclosure were similar to those in Comparative Examples 1 and 2. In addition, it can be seen that, after 4 weeks at a temperature of 40±2°C and a relative humidity of 75±5%, the contents of one recombinant human VEGF receptor extracellular domain 1, one recombinant human VEGF receptor extracellular domain 2 and a recombinant human IgG Fc region (1VEGF1 + 1VEGF2 + Fc) in the pharmaceutical formulations of Examples 2 to 9 were similar to those in Comparative Examples 1 and 2. However, it was confirmed that, at pH 5.5, the content of one recombinant human VEGF receptor extracellular domain 1, one recombinant human VEGF receptor extracellular domain 2 and a recombinant human IgG Fc region (1VEGF1 + 1VEGF2 + Fc) in Example 1 was somewhat disadvantageous compared to those in Comparative Examples 1 to 2, and thus it was necessary to adjust the target pH and pH range to slightly increased values. The experimental results of adjusting the target pH and the pH range are included in Experimental Example 2.

**[Table 7] Number of sub-visible particles (≥2.00 µm, <400.00 µm, particles/mL) measured by HIAC**

| | **After 0 week at 5±3°C** | **After 2 weeks at 5±3°C** | **After 4 weeks at 5±3°C** | **After 2 weeks at 40±2°C and 75±5%** | **After 4 weeks at 40±2°C and 75±5%** |
|---|---|---|---|---|---|
| **Example 1** | 18 | 170 | 115 | 112 | 77 |
| **Example 2** | 88 | 150 | 53 | 62 | 70 |
| **Example 3** | 30 | 20 | 13 | 15 | 78 |
| **Example 4** | 15 | 25 | 12 | 43 | 20 |
| **Example 5** | 105 | 28 | 12 | 60 | 87 |
| **Example 6** | 53 | 42 | 7 | 38 | 52 |
| **Example 7** | 33 | 28 | 7 | 53 | 55 |
| **Example 8** | 8 | 40 | 7 | 28 | 68 |
| **Example 9** | 33 | 68 | 13 | 142 | 73 |
| **Comparative Example 1** | 53 | 72 | 12 | 57 | 48 |
| **Comparative Example 2** | - | - | - | - | - |

Referring to Table 7 above, it can be seen that, after 4 weeks at a temperature of 5±3°C, the numbers of sub-visible particles (≥2.00 µm, <400.00 µm) measured by HIAC in the pharmaceutical formulations of all Examples 1 to 9 satisfying the ranges specified in the present disclosure were similar to that in Comparative Example 1, suggesting that these formulations were very clean pharmaceutical formulations. In addition, it can be seen that, even after 4 weeks at a temperature of 40±2°C and a relative humidity of 75±5%, the numbers of sub-visible particles (≥2.00 µm, <400.00 µm) measured by HIAC in the pharmaceutical formulations of Examples 1 to 9 were similar to that in Comparative Example 1.

**[Table 8] Number of sub-visible particles (≥10.00 µm, <400.00 µm, particles/mL) measured by HIAC**

| | **After 0 week at 5±3°C** | **After 2 weeks at 5±3°C** | **After 4 weeks at 5±3°C** | **After 2 weeks at 40±2°C and 75±5%** | **After 4 weeks at 40±2°C and 75±5%** |
|---|---|---|---|---|---|
| **Example 1** | 5 | 10 | 3 | 23 | 8 |
| **Example 2** | 5 | 23 | 13 | 7 | 2 |
| **Example 3** | 7 | 2 | 0 | 3 | 15 |
| **Example 4** | 2 | 2 | 0 | 17 | 3 |
| **Example 5** | 7 | 7 | 0 | 10 | 10 |
| **Example 6** | 10 | 7 | 0 | 10 | 20 |
| **Example 7** | 25 | 2 | 0 | 13 | 3 |
| **Example 8** | 0 | 8 | 0 | 5 | 3 |
| **Example 9** | 5 | 7 | 0 | 13 | 2 |
| **Comparative Example 1** | 3 | 12 | 0 | 8 | 3 |
| **Comparative Example 2** | - | - | - | - | - |

Referring to Table 8 above, it can be seen that, after 4 weeks at a temperature of 5±3°C, the numbers of sub-visible particles (≥ 10.00 µm, <400.00 µm) measured by HIAC in the pharmaceutical formulations of all Examples 1 to 9 satisfying the ranges specified in the present disclosure were similar to that in Comparative Example 1, suggesting that these formulations were very clean pharmaceutical formulations. In addition, it can be seen that, even after 4 weeks at a temperature of 40±2°C and a relative humidity of 75±5%, the numbers of sub-visible particles (≥ 10.00 µm, <400.00 µm) measured by HIAC in the pharmaceutical formulations of Examples 1 to 9 were similar to that in Comparative Example 1.

**[Table 9] Number of sub-visible particles (≥25.00 µm, <400.00 µm, particles/mL) measured by HIAC**

| | **After 0 week at 5±3°C** | **After 2 weeks at 5±3°C** | **After 4 weeks at 5±3°C** | **After 2 weeks at 40±2°C and 75±5%** | **After 4 weeks at 40±2°C and 75±5%** |
|---|---|---|---|---|---|
| **Example 1** | 2 | 0 | 0 | 2 | 0 |
| **Example 2** | 0 | 5 | 0 | 0 | 0 |
| **Example 3** | 0 | 0 | 0 | 2 | 0 |
| **Example 4** | 0 | 0 | 0 | 2 | 0 |
| **Example 5** | 0 | 3 | 0 | 0 | 0 |
| **Example 6** | 3 | 0 | 0 | 0 | 2 |
| **Example 7** | 5 | 0 | 0 | 2 | 0 |
| **Example 8** | 0 | 2 | 0 | 0 | 0 |
| **Example 9** | 0 | 3 | 0 | 0 | 0 |
| **Comparative Example 1** | 0 | 0 | 0 | 0 | 0 |
| **Comparative Example 2** | - | - | - | - | - |

Referring to Table 9 above, it can be seen that, after 4 weeks at a temperature of 5±3°C, the numbers of sub-visible particles (≥ 25.00 µm, <400.00 µm) measured by HIAC in the pharmaceutical formulations of all Examples 1 to 9 satisfying the ranges specified in the present disclosure were similar to that in Comparative Example 1, suggesting that these formulations were very clean pharmaceutical formulations. In addition, it can be seen that, even after 4 weeks at a temperature of 40±2°C and a relative humidity of 75±5%, the numbers of sub-visible particles (≥25.00 µm, <400.00 µm) measured by HIAC in the pharmaceutical formulations of Examples 1 to 9 were similar to that in Comparative Example 1.

**[Table 10] Number of sub-visible particles (≥1.00 µm, <100.00 µm, particles/mL) measured by MFI**

| | **After 0 week at 5±3°C** | **After 2 weeks at 5±3°C** | **After 4 weeks at 5±3°C** | **After 2 weeks at 40±2°C and 75±5%** | **After 4 weeks at 40±2°C and 75±5%** |
|---|---|---|---|---|---|
| **Example 1** | - | 2596 | - | 2885 | 6418 |
| **Example 2** | 8406 | 1116 | 1906 | 1208 | 6484 |
| **Example 3** | 5255 | 7519 | 2436 | 1678 | 3768 |
| **Example 4** | 14701 | - | 3744 | 514 | 8558 |
| **Example 5** | 16433 | 1367 | 3819 | 1438 | 3190 |
| **Example 6** | 5135 | 990 | 4996 | 1979 | 2812 |
| **Example 7** | 6262 | 566 | 3459 | 1666 | 18324 |
| **Example 8** | 7976 | 5378 | 4145 | 1559 | 4109 |
| **Example 9** | 8554 | 672 | 4055 | 1609 | 11022 |
| **Comparative Example 1** | 6996 | 371 | 3197 | 496 | 3999 |
| **Comparative Example 2** | - | - | - | - | - |

Referring to Table 10 above, it can be seen that, after 4 weeks at a temperature of 5±3°C, the numbers of sub-visible particles (≥ 1.00 µm, <100.00 µm) measured by MFI in the pharmaceutical formulations of all Examples 1 to 9 satisfying the ranges specified in the present disclosure were similar to that in Comparative Example 1, suggesting that these formulations were very clean pharmaceutical formulations. In addition, it can be seen that, even after 4 weeks at a temperature of 40±2°C and a relative humidity of 75±5%, the numbers of sub-visible particles (≥ 1.00 µm, <100.00 µm) measured by MFI in the pharmaceutical formulations of Examples 1 to 9 were similar to that in Comparative Example 1.

**[Table 11] Number of sub-visible particles (≥10.00 µm, <100.00 µm, particles/mL) measured by MFI**

| | **After 0 week at 5±3°C** | **After 2 weeks at 5±3°C** | **After 4 weeks at 5±3°C** | **After 2 weeks at 40±2°C and 75±5%** | **After 4 weeks at 40±2°C and 75±5%** |
|---|---|---|---|---|---|
| **Example 1** | - | 126 | - | 31 | 35 |
| **Example 2** | 22 | 5 | 22 | 10 | 51 |
| **Example 3** | 51 | 27 | 14 | 20 | 33 |
| **Example 4** | 13 | - | 26 | 8 | 48 |
| **Example 5** | 17 | 9 | 19 | 15 | 18 |
| **Example 6** | 19 | 26 | 40 | 31 | 51 |
| **Example 7** | 19 | 7 | 18 | 22 | 40 |
| **Example 8** | 26 | 17 | 34 | 20 | 21 |
| **Example 9** | 22 | 18 | 30 | 96 | 68 |
| **Comparative Example 1** | 9 | 13 | 31 | 4 | 19 |
| **Comparative Example 2** | - | - | - | - | - |

Referring to Table 11 above, it can be seen that, after 4 weeks at a temperature of 5±3°C, the numbers of sub-visible particles (≥ 10.00 µm, <100.00 µm) measured by MFI in the pharmaceutical formulations of all Examples 1 to 9 satisfying the ranges specified in the present disclosure were similar to that in Comparative Example 1, suggesting that these formulations were very clean pharmaceutical formulations. In addition, it can be seen that, even after 4 weeks at a temperature of 40±2°C and a relative humidity of 75±5%, the numbers of sub-visible particles (≥ 10.00 µm, <100.00 µm) measured by MFI in the pharmaceutical formulations of Examples 1 to 9 were similar to that in Comparative Example 1.

**[Table 12] Number of sub-visible particles (≥25.00 µm, <100.00 µm, particles/mL) measured by MFI**

| | **After 0 week at 5±3°C** | **After 2 weeks at 5±3°C** | **After 4 weeks at 5±3°C** | **After 2 weeks at 40±2°C and 75±5%** | **After 4 weeks at 40±2°C and 75±5%** |
|---|---|---|---|---|---|
| **Example 1** | - | 5 | - | 11 | 0 |
| **Example 2** | 4 | 0 | 0 | 0 | 9 |
| **Example 3** | 26 | 3 | 4 | 3 | 9 |
| **Example 4** | 0 | - | 11 | 4 | 8 |
| **Example 5** | 3 | 0 | 2 | 2 | 2 |
| **Example 6** | 3 | 6 | 2 | 4 | 15 |
| **Example 7** | 0 | 0 | 4 | 7 | 7 |
| **Example 8** | 5 | 2 | 4 | 0 | 0 |
| **Example 9** | 7 | 4 | 6 | 7 | 6 |
| **Comparative Example 1** | 0 | 3 | 2 | 0 | 0 |
| **Comparative Example 2** | - | - | - | - | - |

Referring to Table 12 above, it can be seen that, after 4 weeks at a temperature of 5±3°C, the numbers of sub-visible particles (≥25.00 µm, <100.00 µm) measured by MFI in the pharmaceutical formulations of all Examples 1 to 9 satisfying the ranges specified in the present disclosure were similar to that in Comparative Example 1, suggesting that these formulation were very clean pharmaceutical formulations. In addition, it can be seen that, even after 4 weeks at a temperature of 40±2°C and a relative humidity of 75±5%, the numbers of sub-visible particles (≥25.00 µm, <100.00 µm) measured by MFI in the pharmaceutical formulations of Examples 1 to 9 were similar to that in Comparative Example 1.

**[Table 13] Oxidation rate (Met192, %)**

| | **After 0 week at 5±3°C** | **After 2 weeks at 5±3°C** | **After 4 weeks at 5±3°C** | **After 2 weeks at 40±2°C and 75±5%** | **After 4 weeks at 40±2°C and 75±5%** |
|---|---|---|---|---|---|
| **Example 1** | 4.9 | 3.5 | 5.1 | 4.0 | 6.1 |
| **Example 2** | 5.6 | 4.0 | 4.9 | 4.0 | 4.9 |
| **Example 3** | 3.8 | 4.6 | 4.1 | 4.1 | 4.9 |
| **Example 4** | 4.7 | 4.3 | 6.5 | 4.4 | 4.5 |
| **Example 5** | 4.4 | 3.8 | 4.4 | 4.6 | 4.6 |
| **Example 6** | 4.9 | 3.7 | 3.8 | 5.5 | 5.1 |
| **Example 7** | 5.2 | 4.7 | 3.7 | 6.6 | 4.7 |
| **Example 8** | 4.2 | 5.5 | 5.3 | 4.2 | 4.6 |
| **Example 9** | 4.1 | 4.7 | 5.3 | 4.5 | 3.9 |
| **Comparative Example 1** | 4.2 | 3.8 | 4.3 | 4.3 | 5.8 |
| **Comparative Example 2** | 4.6 | 3.2 | 3.7 | 4.0 | 4.4 |

Referring to Table 13 above, it can be seen that, after 4 weeks at a temperature of 5±3°C, the oxidation rates (Met192) of the pharmaceutical formulations of all Examples 1 to 9 satisfying the ranges specified in the present disclosure were similar to those of Comparative Examples 1 and 2. In addition, it can be seen that, even after 4 weeks at a temperature of 40±2°C and a relative humidity of 75±5%, the oxidation rates (Met192) of the pharmaceutical formulations of Examples 1 to 9 were similar to those of Comparative Examples 1 and 2.

**[Table 14] Charge variants (relative content (%) of peak 4 to peak 11 in a total of 12 peaks)**

| | **After 0 week at 5±3°C** | **After 2 weeks at 5±3°C** | **After 4 weeks at 5±3°C** | **After 2 weeks at 40±2°C and 75±5%** | **After 4 weeks at 40±2°C and 75±5%** |
|---|---|---|---|---|---|
| **Example 1** | 87.3 | 86.4 | 87.1 | 86.3 | 88.3 |
| **Example 2** | 90.7 | 87.0 | 87.0 | 86.4 | 86.4 |
| **Example 3** | 87.4 | 87.1 | 87.3 | 86.0 | 85.3 |
| **Example 4** | 89.6 | 86.9 | 87.2 | 86.6 | 87.1 |
| **Example 5** | 87.9 | 86.8 | 86.7 | 86.6 | 86.8 |
| **Example 6** | 87.7 | 86.7 | 86.9 | 86.5 | 85.4 |
| **Example 7** | 88.8 | 86.8 | 87.2 | 86.7 | 86.6 |
| **Example 8** | 88.6 | 86.9 | 87.0 | 86.9 | 87.2 |
| **Example 9** | 88.7 | 86.7 | 87.3 | 86.8 | 86.5 |
| **Comparative Example 1** | 88.9 | 87.0 | 86.6 | 86.7 | 86.2 |
| **Comparative Example 2** | 87.5 | 91.0 | 91.3 | 91.3 | 89.9 |

Referring to Table 14 above, it can be seen that, after 4 weeks at a temperature of 5±3°C, charge variants (the relative content of peak 4 to peak 11 in a total of 12 peaks) in the pharmaceutical formulations of all Examples 1 to 9 satisfying the ranges specified in the present disclosure were similar to those in Comparative Examples 1 and 2. In addition, it can be seen that, even after 4 weeks at a temperature of 40±2°C and a relative humidity of 75±5%, charge variants (the relative content of peak 4 to peak 11 in a total of 12 peaks) in the pharmaceutical formulations of Examples 1 to 9 were similar to those in Comparative Examples 1 and 2.

**[Table 15] VEGF binding affinity (%)**

| | **After 0 week at 5±3°C** | **After 2 weeks at 5±3°C** | **After 4 weeks at 5±3°C** | **After 2 weeks at 40±2°C and 75±5%** | **After 4 weeks at 40±2°C and 75±5%** |
|---|---|---|---|---|---|
| **Example 1** | 82 | - | 86 | 82 | 59 |
| **Example 2** | 89 | - | 78 | 70 | 73 |
| **Example 3** | 91 | - | 79 | 70 | 74 |
| **Example 4** | 98 | - | 72 | 70 | 69 |
| **Example 5** | 90 | - | 82 | 75 | 75 |
| **Example 6** | 95 | - | 81 | 84 | 74 |
| **Example 7** | 89 | - | 83 | 76 | 63 |
| **Example 8** | 99 | - | 73 | 68 | 59 |
| **Example 9** | 96 | - | 81 | 82 | 60 |
| **Comparative Example 1** | 99 | - | 89 | 78 | 78 |
| **Comparative Example 2** | 97 | - | 92 | 79 | 78 |

Referring to Table 15 above, it can be seen that, after 4 weeks at a temperature of 5±3°C, the VEGF binding affinities of the pharmaceutical formulations of all Examples 1 to 9 satisfying the ranges specified in the present disclosure were similar to those of Comparative Examples 1 and 2. In addition, it can be seen that, even after 4 weeks at a temperature of 40±2°C and a relative humidity of 75±5%, the VEGF binding affinities of the pharmaceutical formulations of Examples 1 to 9 were similar to those of Comparative Examples 1 and 2.

### Experimental Example 2: Adjustment of Target pH and pH Range for Improving Low-Molecular-Weight Components Content

For preparation of pharmaceutical formulations to be used in Experimental Example 2, each buffer was prepared so as to have a predetermined pH, and trehalose and sodium chloride were added thereto. Then, a recombinant fusion protein was added thereto and a surfactant was added, thereby preparing the samples shown in Table 16 below. The specific content of each component is shown in Table 16. The following Examples were samples to which aflibercept purified at the Celltrion Research Institute was added, and Comparative Example 1 was a sample to which Regeneron's Eylea (aflibercept) was added. The difference between Example 2 and Example 10 was the protein concentration, and the difference between Examples 10 to 14 was pH. The target volume of each sample for filling a glass vial was 0.278 ml.

The pharmaceutical formulations prepared according to Examples 2 and 10 to 14 and Comparative Example 1 were stored at a temperature of 5±3°C and at a temperature of 50±2°C, and measured for their stabilities after 0 day and 10 days at a temperature of 5±3°C, and for their stabilities after 5 days and 10 days at a temperature of 50±2°C. The results of the measurement are shown in Tables 17 to 30 below.

**[Table 16] Constitution of Experimental Example 2**

| | **Buffer** | **pH** | **Sugar** | **Isotonic agent** | **Surfactant** | **Protein concentration** |
|---|---|---|---|---|---|---|
| **Example 2** | 8.0 mM histidine | 6.0 | 10% (w/v) trehalose | 13.0 mM sodium chloride | 0.03% (w/v) polysorbate 20 | 40 mg/mL |
| **Example 10** | 8.0 mM histidine | 6.0 | 10% (w/v) trehalose | 13.0 mM sodium chloride | 0.03% (w/v) polysorbate 20 | 30 mg/mL |
| **Example 11** | 8.0 mM histidine | 5.5 | 10% (w/v) trehalose | 13.0 mM sodium chloride | 0.03% (w/v) polysorbate 20 | 30 mg/mL |
| **Example 12** | 8.0 mM histidine | 5.9 | 10% (w/v) trehalose | 13.0 mM sodium chloride | 0.03% (w/v) polysorbate 20 | 30 mg/mL |
| **Example 13** | 8.0 mM histidine | 6.2 | 10% (w/v) trehalose | 13.0 mM sodium chloride | 0.03% (w/v) polysorbate 20 | 30 mg/mL |
| **Example 14** | 8.0 mM histidine | 6.5 | 10% (w/v) trehalose | 13.0 mM sodium chloride | 0.03% (w/v) polysorbate 20 | 30 mg/mL |
| **Comparative Example 1** | 10 mM sodium phosphate | 6.2 | 5% (w/v) sucrose | 40.0 mM sodium chloride | 0.03% (w/v) polysorbate 20 | 40 mg/mL |

**[Table 17] Intact main component content (main peak %)**

| | **After 0 day at 5±3°C** | **After 10 days at 5±3°C** | **After 5 days at 50±2°C** | **After 10 days at 50±2°C** |
|---|---|---|---|---|
| **Example 2** | 99.13 | 99.05 | 49.23 | 28.78 |
| **Example 10** | 99.15 | 99.08 | 54.71 | 34.78 |
| **Example 11** | 99.16 | 99.05 | 53.66 | 34.20 |
| **Example 12** | 99.16 | 98.97 | 51.06 | 32.47 |
| **Example 13** | 97.26 | 98.98 | 53.02 | 34.74 |
| **Example 14** | 99.12 | 98.99 | 51.82 | 33.02 |
| **Comparative Example 1** | 98.00 | 97.82 | 36.13 | 21.64 |

Referring to Table 17 above, it can be seen that, after 10 days at a temperature of 5±3°C, the main component contents of the pharmaceutical formulations of Examples 2 and 10 to 14 satisfying the ranges specified in the present disclosure were similar to that of Comparative Example 1. However, it can be seen that, after 10 days at a temperature of 50±2°C, the main component contents of the pharmaceutical formulations of Examples 2 and 10 to 14 were higher than that of Comparative Example 1, suggesting that the formulation of the present disclosure is an improved pharmaceutical formulation capable of more stably storing a desired recombinant fusion protein.

**[Table 18] Content (%) of high-molecular-weight components**

| | **After 0 day at 5±3°C** | **After 10 days at 5±3°C** | **After 5 days at 50±2°C** | **After 10 days at 50±2°C** |
|---|---|---|---|---|
| **Example 2** | 0.84 | 0.92 | 44.81 | 64.84 |
| **Example 10** | 0.75 | 0.90 | 39.99 | 59.85 |
| **Example 11** | 0.81 | 0.90 | 39.27 | 57.98 |
| **Example 12** | 0.83 | 0.99 | 43.80 | 62.17 |
| **Example 13** | 2.70 | 0.98 | 42.91 | 61.49 |
| **Example 14** | 0.86 | 0.99 | 45.23 | 63.92 |
| **Comparative Example 1** | 1.98 | 2.15 | 61.70 | 76.09 |

Referring to Table 18 above, it can be seen that, after 10 days at a temperature of 5±3°C, the high-molecular-weight component contents of the pharmaceutical formulations of Examples 2 and 10 to 14 satisfying the ranges specified in the present disclosure were lower than to that of Comparative Example 1. In addition, it can be seen that, even after 10 days at a temperature of 50±2°C, the high-molecular-weight component contents of the pharmaceutical formulations of Examples 2 and 10 to 14 were lower than to that of Comparative Example 1, suggesting that the formulation of the present disclosure is an improved pharmaceutical formulation capable of more stably storing a desired recombinant fusion protein.

**[Table 19] Content (%) of low-molecular-weight components**

| | **After 0 day at 5±3°C** | **After 10 days at 5±3°C** | **After 5 days at 50±2°C** | **After 10 days at 50±2°C** |
|---|---|---|---|---|
| **Example 2** | 0.04 | 0.03 | 5.96 | 6.38 |
| **Example 10** | 0.10 | 0.02 | 5.29 | 5.38 |
| **Example 11** | 0.03 | 0.05 | 7.06 | 7.82 |
| **Example 12** | 0.01 | 0.04 | 5.14 | 5.36 |
| **Example 13** | 0.03 | 0.04 | 4.07 | 3.77 |
| **Example 14** | 0.02 | 0.02 | 2.95 | 3.06 |
| **Comparative Example 1** | 0.02 | 0.02 | 2.17 | 2.28 |

Referring to Table 19 above, it can be seen that, after 10 days at a temperature of 5±3°C, the low-molecular-weight component contents of the pharmaceutical formulations of Examples 2 and 10 to 14 satisfying the ranges specified in the present disclosure were similar to that of Comparative Example 1. However, it can be seen that, after 10 days at a temperature of 50±2°C, the low-molecular-weight component contents of the pharmaceutical formulations of Examples 2 and 10 to 14 were somewhat higher than that of Comparative Example 1. It can be seen that the low-molecular-weight component contents of Examples 11 to 14 were higher as the pH was lower, and the low-molecular-weight component content of Example 11 at pH 5.5 rapidly increased by about 4% or more beyond the acceptable fluctuation level compared to that of Example 13 which is a pharmaceutical formulation having a target pH. Therefore, it can be seen that the pH range of the formulation of the present disclosure is more preferably set to 5.9 to 6.5.

**[Table 20] Content (%) of one recombinant human VEGF receptor extracellular domain 1, one recombinant human VEGF receptor extracellular domain 2 and recombinant human IgG Fc region (1VEGF1 + 1VEGF2 + Fc)**

| | **After 0 day at 5±3°C** | **After 10 days at 5±3°C** | **After 5 days at 50±2°C** | **After 10 days at 50±2°C** |
|---|---|---|---|---|
| **Example 2** | 96.58 | 96.32 | 88.31 | 82.11 |
| **Example 10** | 96.66 | 96.62 | 88.58 | 84.32 |
| **Example 11** | 96.01 | 96.14 | 86.38 | 81.27 |
| **Example 12** | 96.17 | 96.18 | 89.43 | 85.43 |
| **Example 13** | 96.52 | 96.35 | 92.50 | 91.52 |
| **Example 14** | 96.48 | 96.34 | 95.90 | 92.85 |
| **Comparative Example 1** | 97.37 | 96.11 | 95.77 | 94.11 |

Referring to Table 20 above, it can be seen that, after 10 days at a temperature of 5±3°C, the contents of one recombinant human VEGF receptor extracellular domain 1, one recombinant human VEGF receptor extracellular domain 2 and a recombinant human IgG Fc region (1VEGF1 + 1VEGF2 + Fc) in the pharmaceutical formulations of Examples 2, and 10 to 14 satisfying the ranges specified in the present disclosure were similar to that in Comparative Example 1. However, it can be seen that, after 10 days at a temperature of 50±2°C, the contents of one recombinant human VEGF receptor extracellular domain 1, one recombinant human VEGF receptor extracellular domain 2 and a recombinant human IgG Fc region (1VEGF1 + 1VEGF2 + Fc) in the pharmaceutical formulations of Examples 2, and 10 to 14 were somewhat lower than that in Comparative Example 1. It can be seen that the contents of one recombinant human VEGF receptor extracellular domain 1, one recombinant human VEGF receptor extracellular domain 2 and a recombinant human IgG Fc region (1VEGF1 + 1VEGF2 + Fc) in the pharmaceutical formulations of Examples 11 to 14 decreased as the pH decreased. And the low-molecular-weight component content of Example 11 at pH 5.5 rapidly increased by about 10% or more beyond the acceptable fluctuation level compared to that of Example 13 which is a pharmaceutical formulation having a target pH. Therefore, it can be seen that the pH range of the formulation of the present disclosure is more preferably set to 5.9 to 6.5.

**[Table 21] Number of Sub-visible particles (≥ 1.00 µm, <100.00 µm, particles/mL) measured by MFI**

| | **After 0 day at 5±3°C** | **After 10 days at 5±3°C** | **After 5 days at 50±2°C** | **After 10 days at 50±2°C** |
|---|---|---|---|---|
| **Example 2** | 222 | 530 | 324 | 1201 |
| **Example 10** | 546 | 84 | 234 | 430 |
| **Example 11** | 198 | 245 | 1112 | 704 |
| **Example 12** | 146 | 234 | 153 | 290 |
| **Example 13** | 198 | 143 | 125 | 14709 |
| **Example 14** | 157 | 205 | 231 | 430 |
| **Comparative Example 1** | - | - | - | - |

Referring to Table 21 above, it can be seen that, after 10 days at a temperature of 5±3°C, the numbers of sub-visible particles (≥ 1.00 µm, <100.00 µm) measured by MFI in the pharmaceutical formulations of Examples 2, and 10 to 14 satisfying the ranges specified in the present disclosure were absolutely low, suggesting that these formulations were very clean pharmaceutical formulations. In addition, it can be seen that, even after 10 days at a temperature of 50±2°C, the numbers of sub-visible particles (≥ 1.00 µm, <100.00 µm) measured by MFI in the pharmaceutical formulations of Examples 2, and 10 to 14 were absolutely low, suggesting that these formulations were very clean pharmaceutical formulations.

**[Table 22] Number of Sub-visible particles (≥10.00 µm, <100.00 µm, particles/mL) measured by MFI**

| | **After 0 day at 5±3°C** | **After 10 days at 5±3°C** | **After 5 days at 50±2°C** | **After 10 days at 50±2°C** |
|---|---|---|---|---|
| **Example 2** | 0 | 26 | 2 | 4 |
| **Example 10** | 4 | 4 | 9 | 7 |
| **Example 11** | 0 | 8 | 10 | 29 |
| **Example 12** | 6 | 6 | 4 | 2 |
| **Example 13** | 0 | 6 | 5 | 97 |
| **Example 14** | 11 | 37 | 4 | 14 |
| **Comparative Example 1** | - | - | - | - |

Referring to Table 22 above, it can be seen that, after 10 days at a temperature of 5±3°C, the numbers of sub-visible particles (≥ 10.00 µm, <100.00 µm) measured by MFI in the pharmaceutical formulations of Examples 2, and 10 to 14 satisfying the ranges specified in the present disclosure were absolutely low, suggesting that these formulations were very clean pharmaceutical formulations. In addition, it can be seen that, even after 10 days at a temperature of 50±2°C, the numbers of sub-visible particles (≥ 10.00 µm, <100.00 µm) measured by MFI in the pharmaceutical formulations of Examples 2, and 10 to 14 were absolutely low, suggesting that these formulations were very clean pharmaceutical formulations.

**[Table 23] Number of Sub-visible particles (≥25.00 µm, <100.00 µm, particles/mL) measured by MFI**

| | **After 0 day at 5±3°C** | **After 10 days at 5±3°C** | **After 5 days at 50±2°C** | **After 10 days at 50±2°C** |
|---|---|---|---|---|
| **Example 2** | 0 | 2 | 0 | 0 |
| **Example 10** | 0 | 0 | 0 | 6 |
| **Example 11** | 0 | 4 | 2 | 8 |
| **Example 12** | 4 | 0 | 0 | 0 |
| **Example 13** | 0 | 0 | 0 | 10 |
| **Example 14** | 3 | 12 | 2 | 0 |
| **Comparative Example 1** | - | - | - | - |

Referring to Table 23 above, it can be seen that, after 10 days at a temperature of 5±3°C, the numbers of sub-visible particles (≥25.00 µm, <100.00 µm) measured by MFI in the pharmaceutical formulations of Examples 2, and 10 to 14 satisfying the ranges specified in the present disclosure were absolutely low, suggesting that these formulations were very clean pharmaceutical formulations. In addition, it can be seen that, even after 10 days at a temperature of 50±2°C, the numbers of sub-visible particles (≥25.00 µm, <100.00 µm) measured by MFI in the pharmaceutical formulations of Examples 2, and 10 to 14 were absolutely low, suggesting that these formulations were very clean pharmaceutical formulations.

**[Table 24] VEGF binding affinity (%)**

| | **After 0 day at 5±3°C** | **After 10 days at 5±3°C** | **After 5 days at 50±2°C** | **After 10 days at 50±2°C** |
|---|---|---|---|---|
| **Example 2** | 94 | 106 | 45 | 31 |
| **Example 10** | 104 | 110 | 56 | 34 |
| **Example 11** | 103 | 98 | 38 | 20 |
| **Example 12** | 102 | 91 | 67 | 39 |
| **Example 13** | 99 | 117 | 66 | 58 |
| **Example 14** | 113 | 83 | 78 | 59 |
| **Comparative Example 1** | 110 | 86 | 73 | 55 |

Referring to Table 24 above, it can be seen that, after 10 days at a temperature of 5±3°C, the VEGF binding affinities of the pharmaceutical formulations of Examples 2, and 10 to 14 satisfying the ranges specified in the present disclosure were similar to that of Comparative Example 1. However, it can be seen that, after 10 days at a temperature of 50±2°C, the VEGF binding affinities of some of Examples 2, and 10 to 14 were somewhat higher than that of Comparative Example 1. It can be seen that the VEGF binding affinities of Examples 11 to 14 decreased as the pH decreased. In addition, it can be seen that the VEGF binding affinity of the formulation of Example 11 at pH 5.5 rapidly decreased by about 38% or more beyond the acceptable fluctuation level compared to that of Example 13 which is a pharmaceutical formulation having a target pH. Therefore, it can be seen that the pH range of the formulation of the present disclosure is more preferably set to 5.9 to 6.5.

### Experimental Example 3: Additional Comparison for Determining the Concentration Range of Sodium Chloride Isotonic Agent and Confirmation of Superiority of the Formulation of the Present Disclosure

For preparation of pharmaceutical formulations to be used in Experimental Example 3, each buffer was prepared according to each concentration of sodium chloride, and then a recombinant fusion protein was added thereto and a surfactant was added, thereby preparing the samples shown in Table 25 below. The specific content of each component is shown in Table 25. Examples 15 and 16 and Comparative Example 1 were samples to which aflibercept purified at the Celltrion Research Institute was added. The target volume of each sample for filling a glass vial was 0.278 ml.

The pharmaceutical formulations prepared according to Examples 15 and 16 and Comparative Example 1 were stored at a temperature of 5±3°C and at a temperature of 40±2°C and a relative humidity of 75±5%, and measured for their stabilities after 4 weeks at a temperature of 5±3°C, and for their stabilities after 2 weeks and 4 weeks at a temperature of 40±2°C and a relative humidity of 75±5%. The results of the measurement are shown in Tables 26 to 31 below.

**[Table 25] Constitution of Experimental Example 3**

| | **Buffer** | **pH** | **Sugar** | **Isotonic agent** | **Surfactant** | **Protein concentration** |
|---|---|---|---|---|---|---|
| **Example 15** | 8.0 mM histidine | 6.2 | 10% (w/v) trehalose | 0.0 mM sodium chloride | 0.03% (w/v) polysorbate 20 | 40 mg/mL |
| **Example 16** | 8.0 mM histidine | 6.2 | 10% (w/v) trehalose | 7.2 mM sodium chloride | 0.03% (w/v) polysorbate 20 | 40 mg/mL |
| **Comparative Example 1** | 10 mM sodium phosphate | 6.2 | 5% (w/v) sucrose | 40.0 mM sodium chloride | 0.03 %(w/v) polysorbate 20 | 40 mg/mL |

**[Table 26] Turbidity**

| | **After 4 weeks at 5±3°C** | **After 2 weeks at 40±2°C and 75±5%** | **After 4 weeks at 40±2°C and 75±5%** |
|---|---|---|---|
| **Example 15** | 0.0048 | 0.0065 | 0.0052 |
| **Example 16** | 0.0034 | 0.0064 | 0.0127 |
| **Comparative Example 1** | 0.0078 | 0.0095 | 0.0103 |

Referring to Table 26 above, it can be seen that, even after 4 weeks at a temperature of 5±3°C, Examples 15 and 16 containing a low concentration of sodium chloride showed a very low turbidity, suggesting that they were transparent pharmaceutical formulations. In particular, it can be seen that Examples 15 and 16 showed an absorbance of 0.0400 or less even after 4 weeks of storage at a temperature of 40±2°C and a relatively humidity of 75±5%. It can be seen that the turbidities of Examples 15 and 16 were similar to that of the conventional formulation (Comparative Example 1).

**[Table 27] Charge variants (the relative content (%) of peak 4 to peak 11 in a total of 12 peaks)**

| | **After 4 weeks at 5±3°C** | **After 2 weeks at 40±2°C and 75±5%** | **After 4 weeks at 40±2°C and 75±5%** |
|---|---|---|---|
| **Example 15** | 89.9 | - | 86.9 |
| **Example 16** | 89.6 | - | 87.7 |
| **Comparative Example 1** | 90.3 | - | 86.2 |

Referring to Table 27 above, it can be seen that, after 4 weeks at a temperature of 5±3°C, charge variants (the relative content of peak 4 to peak 11 in a total of 12 peaks) in the pharmaceutical formulations of Examples 15 and 16 containing a low concentration of sodium chloride were similar to those in Comparative Example 1. In addition, it can be seen that, even after 4 weeks at a temperature of 40±2°C and a relative humidity of 75±5%, charge variants (the relative content of peak 4 to peak 11 in a total of 12 peaks) in the pharmaceutical formulations of Examples 15 and 16 were similar to those in Comparative Example 1.

**[Table 28] Number of sub-visible particles (≥1.00µm, <100.00 µm, particles/mL) measured by MFI**

| | **After 4 weeks at 5±3°C** | **After 2 weeks at 40±2°C and 75±5%** | **After 4 weeks at 40±2°C and 75±5%** |
|---|---|---|---|
| **Example 15** | 2770 | 1935 | 2184 |
| **Example 16** | 2504 | 985 | 1355 |
| **Comparative Example 1** | 1399 | 6004 | 9586 |

Referring to Table 28 above, it can be seen that, after 4 weeks at a temperature of 5±3°C, the numbers of sub-visible particles (≥ 1.00 µm, <100.00 µm) measured by MFI in the formulations of Examples 15 and 16 containing a low concentration of sodium chloride were similar to that in Comparative Example 1, suggesting that these formulations were very clean pharmaceutical formulations. In addition, it can be seen that, even after 4 weeks at a temperature of 40±2°C and a relative humidity of 75±5%, the numbers of sub-visible particles (≥ 1.00 µm, <100.00 µm) measured by MFI in the formulations of Examples 15 and 16 were absolutely smaller than that in Comparative Example 1, suggesting that these formulations were very clean pharmaceutical formulations.

**[Table 29] Number of sub-visible particles (≥10.00µm, <100.00 µm, particles/mL) measured by MFI**

| | **After 4 weeks at 5±3°C** | **After 2 weeks at 40±2°C and 75±5%** | **After 4 weeks at 40±2°C and 75±5%** |
|---|---|---|---|
| **Example 15** | 77 | 28 | 61 |
| **Example 16** | 64 | 0 | 22 |
| **Comparative Example 1** | 24 | 216 | 731 |

Referring to Table 29 above, it can be seen that, after 4 weeks at a temperature of 5±3°C, the numbers of sub-visible particles (≥ 10.00 µm, <100.00 µm) measured by MFI in the formulations of Examples 15 and 16 containing a low concentration of sodium chloride were similar to that in Comparative Example 1, suggesting that these formulations were very clean pharmaceutical formulations. In addition, it can be seen that, even after 4 weeks at a temperature of 40±2°C and a relative humidity of 75±5%, the numbers of sub-visible particles (≥ 10.00 µm, <100.00 µm) measured by MFI in the formulations of Examples 15 and 16 were absolutely smaller than that in Comparative Example 1, suggesting that these formulations were very clean pharmaceutical formulations.

**[Table 30] Number of sub-visible particles (≥25.00µm, <100.00 µm, particles/mL) measured by MFI**

| | **After 4 weeks at 5±3°C** | **After 2 weeks at 40±2°C and 75±5%** | **After 4 weeks at 40±2°C and 75±5%** |
|---|---|---|---|
| **Example 15** | 9 | 7 | 8 |
| **Example 16** | 6 | 0 | 0 |
| **Comparative Example 1** | 2 | 4 | 7 |

Referring to Table 30 above, it can be seen that, after 4 weeks at a temperature of 5±3°C, the numbers of sub-visible particles (≥25.00 µm, <100.00 µm) measured by MFI in the formulations of Examples 15 and 16 containing a low concentration of sodium chloride were similar to that in Comparative Example 1, suggesting that these formulations were very clean pharmaceutical formulations. In addition, it can be seen that, even after 4 weeks at a temperature of 40±2°C and a relative humidity of 75±5%, the numbers of sub-visible particles (≥25.00 µm, <100.00 µm) measured by MFI in the formulations of Examples 15 and 16 were similar to that in Comparative Example 1, suggesting that these formulations were very clean pharmaceutical formulations.

**[Table 31] VEGF binding affinity (%)**

| | **After 4 weeks at 5±3°C** | **After 2 weeks at 40±2°C and 75±5%** | **After 4 weeks at 40±2°C and 75±5%** |
|---|---|---|---|
| **Example 15** | 105 | 95 | 87 |
| **Example 16** | 101 | 96 | 68 |
| **Comparative Example 1** | 99 | 85 | 72 |

Referring to Table 31 above, it can be seen that, after 4 weeks at a temperature of 5±3°C, the VEGF binding affinities of the pharmaceutical formulations of Examples 15 and 16 containing a low concentration of sodium chloride were similar to that of Comparative Example 1. In addition, it can be seen that, even after 4 weeks after a temperature of 40±2°C and a relative humidity of 75±5%, the VEGF binding affinities of the pharmaceutical formulations of Examples 15 and 16 containing a low concentration of sodium chloride were similar to that of Comparative Example 1.

### Experimental Example 4: Confirmation of Superiority of the Formulation of the Present Disclosure by Evaluation of Long-Term Stability of Example 13

Evaluation of the long-term stability of Example 13 was performed according to the guideline (Guideline Q5C Quality of Biotechnological Products: Stability Testing of Biotechnological or Biological Products and ICH Guideline Q1A (R2): Stability Testing of New Drug Substances and Drug Products) provided by the International Conference on Harmonisation (ICH).

0.278 mL of the stable liquid pharmaceutical formulation of Example 13 prepared by the method of Experimental Example 1 was stored in a sealed container at 5±3°C/ambient relative humidity. The stabilities of the pharmaceutical formulation were measured after 0 month, 3 months, 6 months and 9 months at the above temperature and humidity. Here, the protein concentration was set to 40 mg/mL.

To evaluate the long-term stability of Example 13, appearance analysis, measurement of the protein concentration using SoloVPE, measurement of the main component, high-molecular-weight component and low-molecular-weight component contents using SEC-HPLC, measurement of the content of one recombinant human VEGF receptor extracellular domain 1, one recombinant human VEGF receptor extracellular domain 2 and a recombinant human IgG Fc region (1VEGF + 1VEGF2 + Fc) by reduced CE-SDS, measurement of charge variants (the relative content of peak 4 to peak 11 in a total of 12 peaks) using cIEF, measurement of VEGF binding affinity, and the number of sub-visible particles were performed, and the results are shown in Tables 32 to 41 below.

**[Table 32] Appearance**

| | **0 month at 5±3°C** | **After 3 months at 5±3°C** | **After 6 months at 5±3°C** | **After 9 months at 5±3°C** |
|---|---|---|---|---|
| **Example 13** | Ivory white | Ivory white | Ivory white | Ivory white |

Referring to Table 32 above, it can be seen that the appearance of Example 13 did not change even after 9 months under the long-term storage temperature condition, suggesting that Example 13 was a stable pharmaceutical formulation.

**[Table 33] Protein concentration (mg/ml)**

| | **0 month at 5±3°C** | **After 3 months at 5±3°C** | **After 6 months at 5±3°C** | **After 9 months at 5±3°C** |
|---|---|---|---|---|
| **Example 13** | 39.1 | 39.9 | 39.7 | 42.4 |

Referring to Table 33 above, it can be seen that the protein concentration of Example 13 did not change even after 9 months under the long-term storage temperature condition, suggesting that Example 13 was a stable pharmaceutical formulation.

**[Table 34] High-molecular-weight component content (%)**

| | **0 month at 5±3°C** | **After 3 months at 5±3°C** | **After 6 months at 5±3°C** | **After 9 months at 5±3°C** |
|---|---|---|---|---|
| **Example 13** | 0.42 | 0.72 | 0.48 | 0.63 |

Referring to Table 34 above, it can be seen that the high-molecular-weight component content of Example 13 did not change even after 9 months under the long-term storage temperature condition, suggesting that Example 13 is a stable pharmaceutical formulation.

**[Table 35] Low-molecular-weight component content (%)**

| | **0 month at 5±3°C** | **After 3 months at 5±3°C** | **After 6 months at 5±3°C** | **After 9 months at 5±3°C** |
|---|---|---|---|---|
| **Example 13** | 0.24 | 0.00 | 0.00 | 0.00 |

Referring to Table 35 above, it can be seen that the low-molecular-weight component content of Example 13 did not change even after 9 months under the long-term storage temperature condition, suggesting that Example 13 was a stable pharmaceutical formulation.

**[Table 36] Content (%) of one recombinant human VEGF receptor extracellular domain 1, one recombinant human VEGF receptor extracellular domain 2 and recombinant human IgG Fc region (1VEGF1 + 1VEGF2 + Fc)**

| | **0 month at 5±3°C** | **After 3 months at 5±3°C** | **After 6 months at 5±3°C** | **After 9 months at 5±3°C** |
|---|---|---|---|---|
| **Example 13** | 97.42 | 97.48 | 97.40 | 97.37 |

Referring to Table 36 above, it can be seen that the content (%) of one recombinant human VEGF receptor extracellular domain 1, one recombinant human VEGF receptor extracellular domain 2 and a recombinant human IgG Fc region (1VEGF1 + 1VEGF2 + Fc) in Example 13 did not change even after 9 months under the long-term storage temperature condition, suggesting that Example 13 was a stable pharmaceutical formulation.

**[Table 37] Number of sub-visible particles (≥2.00 µm, <400.00 µm, particles/mL) measured by HIAC**

| | **0 month at 5±3°C** | **After 3 months at 5±3°C** | **After 6 months at 5±3°C** | **After 9 months at 5±3°C** |
|---|---|---|---|---|
| **Example 13** | - | 185 | 172 | 297 |

Referring to Table 37 above, it can be seen that the number of sub-visible particles (≥ 2.00 µm, <400.00 µm) in Example 13 was small even after 9 months under the long-term storage temperature condition, suggesting that Example 13 was a very clean pharmaceutical formulation.

**[Table 38] Number of sub-visible particles (≥10.00 µm, <400.00 µm, particles/mL) measured by HIAC**

| | **0 month at 5±3°C** | **After 3 months at 5±3°C** | **After 6 months at 5±3°C** | **After 9 months at 5±3°C** |
|---|---|---|---|---|
| **Example 13** | 8 | 3 | 8 | 33 |

Referring to Table 38 above, it can be seen that the number of sub-visible particles (≥ 10.00 µm, <400.00 µm) in Example 13 was small even after 9 months under the long-term storage temperature condition, suggesting that Example 13 was a very clean pharmaceutical formulation.

**[Table 39] Number of sub-visible particles (≥25.00 µm, <400.00 µm, particles/mL) measured by HIAC**

| | **0 month at 5±3°C** | **After 3 months at 5±3°C** | **After 6 months at 5±3°C** | **After 9 months at 5±3°C** |
|---|---|---|---|---|
| **Example 13** | 0 | 0 | 0 | 5 |

Referring to Table 39 above, it can be seen that the number of sub-visible particles (≥ 25.00 µm, <400.00 µm) in Example 13 was small even after 9 months under the long-term storage temperature condition, suggesting that Example 13 was a very clean pharmaceutical formulation.

**[Table 40] Charge variants (the relative content (%) of peak 4 to peak 11 in a total of 12 peaks)**

| | **0 month at 5±3°C** | **After 3 months at 5±3°C** | **After 6 months at 5±3°C** | **After 9 months at 5±3°C** |
|---|---|---|---|---|
| **Example 13** | 87.9 | 87.6 | 88.0 | 87.5 |

Referring to Table 40 above, it can be seen that charge variants (the relative content (%) of peak 4 to peak 11 in a total of 12 peaks) in Example 13 did not change even after 9 months under the long-term storage temperature condition, suggesting that Example 13 was a stable pharmaceutical formulation.

**[Table 41] VEGF binding affinity (%)**

| | **0 month at 5±3°C** | **After 3 months at 5±3°C** | **After 6 months at 5±3°C** | **After 9 months at 5±3°C** |
|---|---|---|---|---|
| **Example 13** | 112 | 106 | 98 | 92 |

Referring to Table 41 above, it can be seen that VEGF binding affinity of Example 13 did not change even after 9 months under the long-term storage temperature condition, suggesting that Example 13 was a stable pharmaceutical formulation.

As described above, the stable pharmaceutical formulation according to the present disclosure has low viscosity while containing a recombinant fusion protein, may maintain excellent stability under long-term storage conditions, accelerated conditions and stress conditions, and may be administered intraocularly.

## Claims

1. A stable pharmaceutical formulation containing:
(A) a recombinant fusion protein;
(B) a surfactant;
(C) a sugar or a derivative thereof; and
(D) a buffer.

2. The stable pharmaceutical formulation of claim 1, wherein the stable pharmaceutical formulation is in a liquid form.

3. The stable pharmaceutical formulation of claim 1, wherein the recombinant fusion protein (A) comprises a vascular endothelial growth factor (VEGF) antagonist.

4. The stable pharmaceutical formulation of claim 1, wherein the recombinant fusion protein (A) comprises a human VEGF receptor extracellular domain.

5. The stable pharmaceutical formulation of claim 1, wherein the recombinant fusion protein (A) comprises human VEGF receptor extracellular domain 1, human VEGF receptor extracellular domain 2, or a mixture thereof.

6. The stable pharmaceutical formulation of claim 4 or 5, wherein the recombinant fusion protein (A) comprises a human immunoglobulin G (IgG) Fc region.

7. The stable pharmaceutical formulation of claim 1, wherein the recombinant fusion protein (A) comprise:
(1) a VEGF receptor extracellular domain 1 component comprising amino acids 27 to 129 of SEQ ID NO: 2;
(2) a VEGF receptor extracellular domain 2 component comprising amino acids 130 to 231 of SEQ ID NO: 2; and
(3) an Fc region component comprising amino acids 232 to 457 of SEQ ID NO: 2.

8. The stable pharmaceutical formulation of claim 1, wherein the recombinant fusion protein (A) comprise aflibercept.

9. The stable pharmaceutical formulation of claim 1, wherein the recombinant fusion protein (A) is contained at a concentration of 5 to 100 mg/ml.

10. The stable pharmaceutical formulation of claim 1, wherein the surfactant (B) comprises polysorbate, poloxamer, or a mixture thereof.

11. The stable pharmaceutical formulation of claim 10, wherein the surfactant (B) comprises polysorbate 20, polysorbate 40, polysorbate 60, polysorbate 80, or a mixture of two or more thereof.

12. The stable pharmaceutical formulation of claim 11, wherein the surfactant (B) comprises polysorbate 20.

13. The stable pharmaceutical formulation of claim 1, wherein the surfactant (B) is contained at a concentration of 0.01 to 0.1% (w/v).

14. The stable pharmaceutical formulation of claim 1, wherein the sugar (C) comprises a monosaccharide, a disaccharide, an oligosaccharide, a polysaccharide, or a mixture of two or more thereof, and the derivative of the sugar (C) comprises a sugar alcohol, a sugar acid, or a mixture thereof.

15. The stable pharmaceutical formulation of claim 1, wherein the sugar or the derivative thereof (C) comprises sorbitol, mannitol, trehalose, sucrose, or a mixture of two or more thereof.

16. The stable pharmaceutical formulation of claim 15, wherein the sugar or the derivative thereof (C) comprises trehalose.

17. The stable pharmaceutical formulation of claim 1, wherein the sugar or the derivative thereof (C) is contained at a concentration of 1 to 20% (w/v).

18. The stable pharmaceutical formulation of claim 1, wherein the buffer (D) comprises an amino acid.

19. The stable pharmaceutical formulation of claim 1, wherein the buffer (D) comprises a free amino acid, an amino acid salt, or a mixture thereof.

20. The stable pharmaceutical formulation of claim 19, wherein the buffer (D) comprises histidine, histidine salt, or a mixture thereof.

21. The stable pharmaceutical formulation of claim 1, wherein the buffer (D) is contained at a concentration of 1 to 20 mM.

22. The stable pharmaceutical formulation of claim 1, wherein the stable pharmaceutical formulation is free of acetic acid, citric acid, phosphoric acid, or a mixture thereof.

23. The stable pharmaceutical formulation of claim 1, further containing (E) an isotonic agent.

24. The stable pharmaceutical formulation of claim 23, wherein the isotonic agent (E) comprises sodium chloride, potassium chloride, calcium chloride, or a mixture of two or more thereof.

25. The stable pharmaceutical formulation of claim 23, wherein the isotonic agent (E) is contained at a concentration of 30 mM or less.

26. The stable pharmaceutical formulation of claim 1, wherein the stable pharmaceutical formulation has a pH of 5.0 to 7.0.

27. The stable pharmaceutical formulation of claim 1, wherein the stable pharmaceutical formulation is free of NaF, KBr, NaBr, Na₂SO₄, NaSCN, K₂SO₄, a mixture thereof.

28. The stable pharmaceutical formulation of claim 1, wherein the stable pharmaceutical formulation is free of a chelating agent.

29. A stable pharmaceutical formulation containing:
(A) 5 to 100 mg/ml of a recombinant fusion protein comprising (1) a vascular endothelial growth factor(VEGF) receptor extracellular domain 1 component comprising amino acids 27 to 129 of SEQ ID NO: 2, (2) a VEGF receptor extracellular domain 2 component comprising amino acids 130 to 231 of SEQ ID NO: 2, and (3) an Fc region component comprising amino acids 232 to 457 of SEQ ID NO: 2;
(B) 0.01 to 0.1% (w/v) of a surfactant;
(C) 1 to 20% (w/v) of a sugar or a derivative thereof; and
(D) 1 to 20 mM of a buffer.

30. The stable pharmaceutical formulation of claim 29, further containing (E) 30 mM or less of an isotonic agent.

31. A stable pharmaceutical formulation containing:
(A) a vascular endothelial growth factor (VEGF) antagonist;
(B) polysorbate;
(C) a sugar or a derivative thereof; and
(D) histidine.

32. The stable pharmaceutical formulation of claim 31, further containing (E) sodium chloride.

33. A stable pharmaceutical formulation containing:
(A) 5 to 100 mg/ml of a recombinant fusion protein comprising (1) a vascular endothelial growth factor(VEGF) receptor extracellular domain 1 component comprising amino acids 27 to 129 of SEQ ID NO: 2, (2) a VEGF receptor extracellular domain 2 component comprising amino acids 130 to 231 of SEQ ID NO: 2, and (3) an Fc region component comprising amino acids 232 to 457 of SEQ ID NO:2;
(B) 0.01 to 0.1% (w/v) of polysorbate;
(C) 1 to 20% (w/v) of a sugar or a derivative thereof; and
(D) 1 to 20 mM of histidine.

34. The stable pharmaceutical formulation of claim 33, further containing (E) 30 mM or less of sodium chloride.

35. The stable pharmaceutical formulation of any one of claims 1 to 34, wherein the number of sub-visible particles having a particle diameter of equal to or more than 10.00 µm to less than 400.00 µm is 50 or less as measured by HIAC after 9 months of storage at 5±3°C.

36. The stable pharmaceutical formulation of any one of claims 1 to 34, wherein the stable pharmaceutical formulation shows a main component content of 98% or more after 10 days of storage at 5±3°C.

37. The stable pharmaceutical formulation of any one of claims 1 to 34, wherein the stable pharmaceutical formulation shows a high-molecular-weight component content of 1% or less after 10 days of storage at 5±3°C.

38. The stable pharmaceutical formulation of any one of claims 1 to 34, wherein the stable pharmaceutical formulation shows a low-molecular-weight component content of 0.05% or less after 10 days of storage at 5±3°C.

39. The stable pharmaceutical formulation of any one of claims 1 to 34, wherein the stable pharmaceutical formulation shows a charge variant content of 87% or more after 9 months of storage at 5±3°C.

40. The stable pharmaceutical formulation of any one of claims 1 to 34, wherein the stable pharmaceutical formulation shows a VEGF binding affinity of 90% or more after 9 months of storage at 5±3°C.

41. The stable pharmaceutical formulation of any one of claims 1 to 34, wherein the stable pharmaceutical formulation is for intraocular administration.

42. The stable pharmaceutical formulation of any one of claims 1 to 34, wherein the stable pharmaceutical formulation is not subjected to a reconstitution step, a dilution step, or both, before use.

43. A glass vial filled with the stable pharmaceutical formulation set forth in any one of claims 1 to 34.

44. A pre-filled syringe filled with the stable pharmaceutical formulation set forth in any one of claims 1 to 34.
